(19) Europäisches Patentamt
European Patent Office
Office européen des brevets

(11) EP 1 047 458 B1

(12) EUROPEAN PATENT SPECIFICATION

(45) Date of publication and mention of the grant of the patent:
**08.06.2011 Bulletin 2011/23**

(21) Application number: **99937340.0**

(22) Date of filing: **21.07.1999**

(51) Int Cl.:
***A61L 2/00*** *(2006.01)*

(86) International application number:
**PCT/US1999/016404**

(87) International publication number:
**WO 2000/004930 (03.02.2000 Gazette 2000/05)**

(54) **METHOD FOR INACTIVATION OF MICROORGANISMS USING PHOTOSENSITIZERS**

VERFAHREN ZUR INAKTIVIERUNG VON MIKROORGANISMEN UNTER VERWENDUNG VON PHOTOSENSIBILISATOREN

PROCEDE ET APPAREIL PERMETTANT D'INACTIVER DES CONTAMINANTS BIOLOGIQUES A L'AIDE DE PHOTOSENSIBILISANTS

(84) Designated Contracting States:
**AT BE CH CY DE DK ES FI FR GB GR IE IT LI LU MC NL PT SE**
Designated Extension States:
**AL LT LV MK RO SI**

(30) Priority: **21.07.1998 US 119666**
**20.07.1999 US 357188**

(43) Date of publication of application:
**02.11.2000 Bulletin 2000/44**

(60) Divisional application:
**08006177.3 / 1 972 351**
**10000970.3 / 2 174 669**

(73) Proprietor: **CaridianBCT Biotechnologies, LLC**
**Lakewood CO 80215 (US)**

(72) Inventors:
- **GOODRICH, Raymond, Paul, Jr.**
  **Denver, CO 80209 (US)**
- **CORBIN, Frank, III**
  **Littleton, CO 80123 (US)**
- **WOOD, Edward, C., Jr.**
  **Lakewood, CO 80228 (US)**
- **HLAVINKA, Dennis**
  **Golden, CO 80419 (US)**

(74) Representative: **Benson, John Everett et al**
**J. A. Kemp & Co.**
**14 South Square**
**Gray's Inn**
**London WC1R 5JJ (GB)**

(56) References cited:
**EP-A- 0 196 515** **EP-A- 0 801 072**
**WO-A-88/10087** **WO-A-94/07499**

- **UEHARA K ET AL: "Effect of adenine on the riboflavin-sensitized photoreaction. II. Effect of adenine on the photodynamic inactivation of transforming deoxyribonucleic acid in the presence of riboflavin" THE JOURNAL OF BIOCHEMISTRY, vol. 71, no. 5, 1972, pages 805-810, XP000889634 Tokyo, Japan**
- **REINHARDT A ET AL: "Virucidal activity of retinal" ANTIMICROBIAL AGENTS AND CHEMOTHERAPY, vol. 16, no. 3, September 1979 (1979-09), pages 421-423, XP000884452**
- **MURATA A ET AL: "Effect of vitamins other than vitamin C on viruses: virus-inactivating activity of vitamin K5" JOURNAL OF NUTRITIONAL SCIENCE AND VITAMINOLOGY, vol. 29, no. 6, 1983, pages 721-724, XP000884650**
- **UEHARA K ET AL: "Effect of adenine on the riboflavin-sensitized photoreaction. I. Effect of adenine on the photodynamic inactivation of yeast alcohol dehydrogenase in the presence of riboflavin" THE JOURNAL OF VITAMINOLOGY, vol. 17, no. 3, 1971, pages 148-154, XP000889600 Kyoto, Japan**
- **CHEMICAL ABSTRACTS, vol. 87, no. 1, 2 January 1978 (1978-01-02) Columbus, Ohio, US; abstract no. 400626, KABUTA H ET AL: "Inactivation of viruses by dyes and visible light" XP002122366 & KARUME IGAKKAI ZASSHI, vol. 39, no. 11, 1976, pages 1067-1077,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CHEMICAL ABSTRACTS, vol. 98, no. 1, 3 January 1983 (1983-01-03) Columbus, Ohio, US; abstract no. 1200, KOBAYASHI ET AL: "The molecular mechanism of mutation. Photodynamic action of flavins on the RNA-synthesizing system" XP002122365 & TOKYO IKA DAIGAKU ZASSHI, vol. 40, no. 3, 1982, pages 409-421,**
- **HEMA KALE ET AL: "Assessment of the genotoxic potential of riboflavin and lumiflavin" MUTATION RESEARCH, vol. 298, no. 1, November 1992 (1992-11), pages 17-23, XP002122364**


Remarks:

The file contains technical information submitted after the application was filed and not included in this specification

## Description

Contamination of blood supplies with infectious microorganisms such as HIV, hepatitis and other viruses and bacteria presents a serious health hazard for those who must receive transfusions of whole blood or administration of various blood components such as platelets, red cells, blood plasma, Factor VIII, plasminogen, fibronectin, anti-thrombin III, cryoprecipitate, human plasma protein fraction, albumin, immune serum globulin, prothrombin complex plasma growth hormones, and other components isolated from blood. Blood screening procedures may miss contaminants, and sterilization procedures which do not damage cellular blood components but effectively inactivate all infectious viruses and other microorganisms have not heretofore been available.

Solvent detergent methods of blood component decontamination work by dissolving phospholipid membranes surrounding viruses such as HIV, and do not damage protein components of blood; however, if blood cells are present, such methods cannot be used because of damage to cell membranes.

The use of photosensitizers, compounds which absorb light of a defined wavelength and transfer the absorbed energy to an energy acceptor, has been proposed for blood component sterilization. For example, European Patent application 196.515 published October 8, 1986, suggests the use of non-endogenous photosensitizers such as porphyrins, psoralens, acridine, toluidines, flavine (acriflavine hydrochloride), phenothiazine derivatives, and dyes such as neutral red, and methylene blue, as blood additives. Protoporphyrin, which occurs naturally within the body, can be metabolized to form a photosensitizer; however, its usefulness is limited in that it degrades desired biological activities of proteins. Chlorpromazine, is also exemplified as one such photosensitizer; however its usefulness is limited by the fact that it should be removed from any fluid administered to a patient after the decontamination procedure because it has a sedative effect.

Goodrich, R.P., et al. (1997), "The Design and Development of Selective, Photoactivated Drugs for Sterilization of Blood Products," Drugs of the Future 22:159-171 provides a review of some photosensitizers including psoralens, and some of the issues of importance in choosing photosensitizers for decontamination of blood products. The use of texaphyrins for DNA photocleavage is described in U.S. Patent Nos. 5,607,924 issued March 4, 1997 and 5,714,328 issued February 3, 1998 to Magda et al. The use of sapphyrins for viral deactivation is described in U.S. Patent No. 5,041,078 issued August 20, 1991 to Matthews, et al. Inactivation of extracellular enveloped viruses in blood and blood components by Phenthiazin-5-ium dyes plus light is described in U.S. Patent No. 5,545,516 issued August 13, 1996 to Wagner. The use of porphyrins, hematoporphyrins, and merocyanine dyes as photosensitizing agents for eradicating infectious contaminants such as viruses and protozoa from body tissues such as body fluids is disclosed in U.S. Patent 4,915,683 issued April 10, 1990 and related U.S. Patent No. 5,304,113 issued April 19, 1994 to Sieber et al. The mechanism of action of such photosensitizers is described as involving preferential binding to domains in lipid bilayers, e.g. on enveloped viruses and some virus-infected cells. Photoexcitation of membrane-bound agent molecules leads to the formation of reactive oxygen species such as singlet oxygen which causes lipid peroxidation. A problem with the use of such photosensitizers is that they attack cell membranes of desirable components of fluids to be decontaminated, such as red blood cells, and the singlet oxygen also attacks desired protein components of fluids being treated. U.S. Patent 4,727,027 issued February 23, 1988 to Wiesehahn, G.P., et al. discloses the use of furocoumarins including psoralen and derivatives for decontamination of blood and blood products, but teaches that steps must be taken to reduce the availability of dissolved oxygen and other reactive species in order to inhibit denaturation of biologically active proteins. Photoinactivation of viral and bacterial blood contaminants using halogenated coumarins is described in U.S. Patent 5,516,629 issued May 14, 1996 to Park, et al. U.S. Patent 5,587,490 issued December 24, 1996 to Goodrich Jr., R.P., et al. and U.S. Patent No. 5,418,130 to Platz, et al. disclose the use of substituted psoralens for inactivation of viral and bacterial blood contaminants. The latter patent also teaches the necessity of controlling free radical damage to other blood components. U.S. Patent 5,654,443 issued August 5, 1997 to Wollowitz et al. teaches new psoralen compositions used for photodecontamination of blood. U.S. Patent 5,709,991 issued January 20, 1998 to Lin et al. teaches the use of psoralen for photodecontamination of platelet preparations and removal of psoralen afterward. U.S. Patent 5,120,649 issued June 9, 1992 and related U.S. Patent 5,232,844 issued August 3, 1993 to Horowitz, et al., also disclose the need for the use of "quenchers" in combination with photosensitizers which attack lipid membranes, and U.S. Patent 5,360,734 issued November 1, 1994 to Chapman et al. also addresses this problem of prevention of damage to other blood components.

Photosensitizers which attack nucleic acids are known to the art. U.S. Patent 5,342,752 issued August 30, 1994 to Platz et al. discloses the use of compounds based on acridine dyes to reduce parasitic contamination in blood matter comprising red blood cells, platelets, and blood plasma protein fractions. These materials, although of fairly low toxicity, do have some toxicity e.g. to red blood cells. This patent fails to disclose an apparatus for decontaminating blood on a flow-through basis. U.S. Patent No. 5,798,238 to Goodrich, Jr., et al., discloses the use of quinolone and quinolone compounds for inactivation of viral and bacterial contaminants.

Binding of DNA with photoactive agents has been exploited in processes to reduce lymphocytic populations in blood as taught in U.S. Patent No. 4,612,007 issued September 16, 1986 and related U.S. Patent No. 4,683,889

issued August 4, 1987 to Edelson.

Riboflavin (7,8-dimethyl-10-ribityl isoalloxazine) has been reported to attack nucleic acids. Photoalteration of nucleic acid in the presence of riboflavin is discussed in Tsugita, A, et al. (1965), "Photosensitized inactivation of ribonucleic acids in the presence of riboflavin," Biochimica et Biophysica Acta 103:360-363; and Speck, W.T. et al. (1976), "Further Observations on the Photooxidation of DNA in the Presence of Riboflavin," Biochimica et Biophysica Acta 435: 39-44. Binding of lumiflavin (7,8,10-trimethylisoalloxazine) to DNA is discussed in Kuratomi, K., et al. (1977), "Studies on the Interactions between DNA and Flavins," Biochimica et Biophysica Acta 476:207-217. Hoffmann, M.E., et al. (1979), "DNA Strand Breaks in Mammalian Cells Exposed to Light in the Presence of Riboflavin and Tryptophan," Photochemistry and Photobiology 29:299-303 describes the use of riboflavin and tryptophan to induce breaks in DNA of mammalian cells after exposure to visible fluorescent light or near-ultraviolet light. The article states that these effects did not occur if either riboflavin or tryptophan was omitted from the medium. DNA strand breaks upon exposure to proflavine and light are reported in Piette, J. et al. (1979), "Production of Breaks in Single- and Double-Stranded Forms of Bacteriophage ΦX174 DNA by Proflavine and Light Treatment," Photochemistry and Photobiology 30:369-378, and alteration of guanine residues during proflavine-mediated photosensitization of DNA is discussed in Piette, J., et al. (1981), "Alteration of Guanine Residues during Proflavine Mediated Photosensitization of DNA," Photochemistry and Photobiology 33:325-333.

J. Cadet, et al. (1983), "Mechanisms and Products of Photosensitized Degradation of Nucleic Acids and Related Model Compounds," Israel J. Chem. 23:420-429, discusses the mechanism of action by production of singlet oxygen of rose bengal, methylene blue, thionine and other dyes, compared with mechanisms not involving production of singlet oxygen by which nucleic acid attack by flavin or pteron derivatives proceeds. Riboflavin is exemplified in this disclosure as having the ability to degrade nucleic acids. Korycka-Dahl, M., et al. (1980), "Photodegradation of DNA with Fluorescent Light in the Presence of Riboflavin, and Pholoprotection by Flavin Triplet-State Quenchers," Biochimica et Biophysica Acta 610:229-234 also discloses that active oxygen species are not directly involved in DNA scission by riboflavin. Peak, J.G., et al. (1984), "DNA Breakage Caused by 334-nm Ultraviolet Light is Enhanced by Naturally Occurring Nucleic Acid Components and Nucleotide Coenzymes," Photochemistry and Photobiology 39:713-716 further explores the mechanism of action of riboflavin and other photosensitizers. However, no suggestion is made that such photosensitizers be used for decontamination of medical fluids.

Apparatuses for decontamination of blood have been describe in U.S. Patent No. 5,290,221 issued March 1, 1994 to Wolfe, Jr., et al. and U.S. Patent No. 5,536,238 issued July 16, 1996 to Bischof. U.S. Patent No. 5,290,221 discloses the irradiation of fluid in a relatively narrow, arcuate gap. U.S. Patent 5,536,238 discloses devices utilizing optical fibers extending into a filtration medium. Both patents recommend as photosensitizers benzoporphryin derivatives which have an affinity for cell walls.

## SUMMARY

A method is provided for treating a fluid or other material to inactivate at least some of the microorganisms and white cells which may be present therein or thereon.

The invention provides a method for treating a fluid *in vitro* to inactive microorganisms which may be present therein, wherein said fluid contains one or more components selected from whole blood, biologically active proteins derived from blood or blood constituents, red blood cells, platelets, plasma and therapeutic protein compositions containing proteins derived from blood, said method comprising:

(a) adding an inactivation-effective amount of a photosensitizer which is an endogenous alloxazine or endogenously-based alloxazine derivative to said fluid;
(b) exposing the fluid of step (a) to ultraviolet light, whereby said microorganisms are inactivated.

One mechanism by which these photosensitizers may inactivate microorganisms is by interfering with nucleic acids, so as to prevent replication of the nucleic acid.

As used herein, the term "inactivation of a microorganism" means totally or partially preventing the microorganism from replicating, either by killing the microorganism or otherwise interfering with its ability reproduce.

Microorganisms include viruses (both extracellular and intracellular), bacteria, bacteriophages, fungi, blood-transmitted parasites, and protozoa. Exemplary viruses include acquired immunodeficiency (HIV) virus, hepatitis A, B and C viruses, sinbis virus, cytomegalovirus, vesicular stomatitis virus, herpes simplex virus, e.g. types I and II, human T-lymphotropic retroviruses, HTLV-III, lymphadenopathy virus LAV/IDAV, parvovirus, transfusion-transmitted (TT) virus, Epstein-Barr virus, and others known to the art. Bacteriophages include ΦX174, Φ6, λ, R17, $T_4$, and $T_2$.
Exemplary bacteria include *P. aeruginosa, S. aureus, S. epidermis, L. monocytogenes, E. coli, K. pneumonia* and *S. marcescens.*

Inactivation of white blood cells may be desirable when suppression of immune or autoimmune response is

desired, e.g., in processes involving transfusion of red cells, platelets or plasma when donor white blood cells may be present.

Materials which are treated by the methods of this invention are adequately permeable to photoradiation to provide sufficient light to achieve viral inactivation, or which can be suspended or dissolved in fluids which have such permeability to photoradiation. The materials to be treated are whole blood and aqueous compositions containing biologically active proteins derived from blood or blood constituents, red cells, platelets, plasma (fresh or fresh frozen plasma) and therapeutic protein compositions containing proteins derived from blood, such as fluids containing biologically active protein useful in the treatment of medical disorders, e.g. factor VIII, Von Willebrand factor, factor IX, factor X, factor XI, Hageman factor, prothrombin, anti-thrombin III, fibronectin, plasminogen, plasma protein fraction, immune serum globulin, modified immune globulin, albumin, plasma growth hormone, somatomedin, plasminogen streptokinase complex, ceruloplasmin, transferrin, haptoglobin, antitrypsin and prekallikrein.

The term "biologically active" means capable of effecting a change in a living organism or component thereof. "Biologically active" with respect to "biologically active protein" as referred to herein does not refer to proteins which are part of the microorganisms being inactivated. Similarly, "non-toxic" with respect to the photosensitizers means low or no toxicity to humans and other mammals, and does not mean non-toxic to the microorganisms being inactivated. "Substantial destruction" of biological activity means at least as much destruction as is caused by porphyrin and porphyrin derivatives, metabolites and precursors which are known to have a damaging effect on biologically active proteins and cells of humans and mammals. Similarly, "substantially non-toxic" means less toxic than porphyrin, porphyrin derivatives, metabolites and precursors that are known for blood sterilization.

The term "blood product" as used herein includes blood constituents and therapeutic protein compositions containing proteins derived from blood as defined above. Fluids containing biologically active proteins other than those derived from blood may also be treated by the methods of this invention.

Decontamination methods of this invention using endogenous photosensitizers and endogenously-based photosensitizer derivatives do not substantially destroy the biological activity of fluid components other than microorganisms. As much biological activity of these components as possible is retained, although in certain instances, when the methods are optimized, some loss of biological activity, e.g., denaturization of protein components, must be balanced against effective decontamination of the fluid. So long as fluid components retain sufficient biological activity to be useful for their intended or natural purposes, their biological activities are not considered to be "substantially destroyed."

The photosensitizers used in this invention are endogenous alloxazines or endogenously based alloxazine derivatives. A "photosensitizer" is defined as any' compound which absorbs radiation of one or more defined wavelengths and subsequently utilizes the absorbed energy to carry out a chemical process. The photosensitizers used in this invention preferentially adsorb to nucleic acids, thus focusing their photodynamic effect upon microorganisms and viruses with little or no effect upon accompanying cells or proteins. The term "endogenous" means naturally found in a human or mammalian body, either as a result of synthesis by the body or because of ingestion as an essential foodstuff (e.g. vitamins) or formation of metabolites and/or byproducts *in vivo*. Examples of endogenous alloxazine photosensitizers are 7,8-dimethyl-10-ribityl isoalloxazine (riboflavin), 7,8,10-trimethylisoalloxazine (lumiflavin), 7,8-dimethylalloxazine (lumichrome), isoalloxazine-adenine dinucleotide (flavine adenine dinucleotide [FAD]) and alloxazine mononucleotide (also known as flavine mononucleotide [FMN] and riboflavine-5-phosphate). The term "alloxazine" includes isoalloxazines, Endogenously-based derivative photosensitizers include synthetically derived analogs and homologs of endogenous photosensitizers which may have or lack lower (1-5) alkyl or halogen substituents of the photosensitizers from which they are derived, and which preserve the function and substantial non-toxicity thereof. When endogenous photosensitizers are used, particularly when such photosensitizers are not inherently toxic or do not yield toxic photoproducts after photoradiation, no removal or purification step is required after decontamination, and treated product can be directly returned to a patient's body or administered to a patient in need of its therapeutic effect. Preferred endogenous alloxazine photosensitizers are:

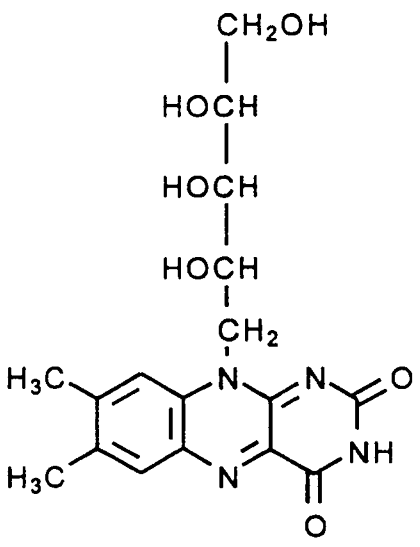

7,8-dimethyl-10-ribityl isoalloxazine

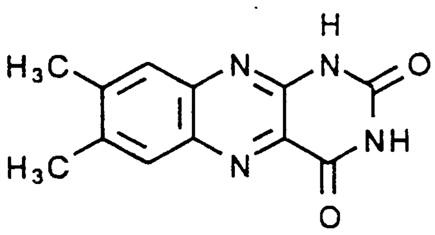

7,8-dimethylalloxazine

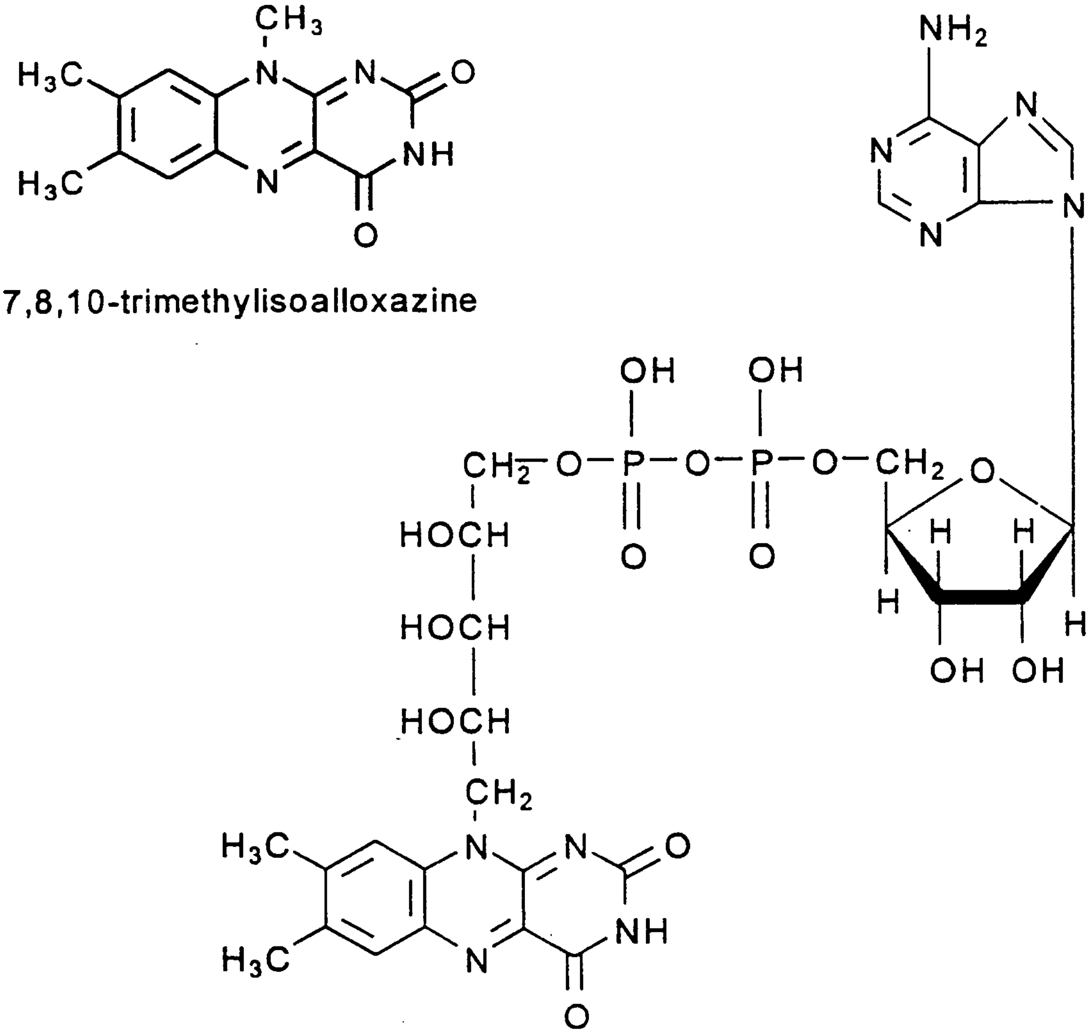

7,8,10-trimethylisoalloxazine

Isoalloxazine-adenine dinucleotide

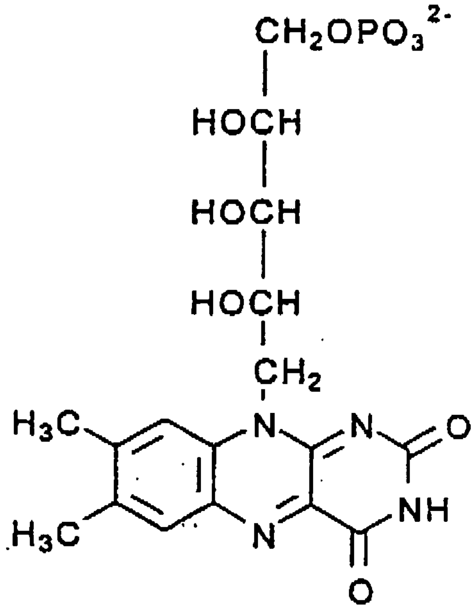

Alloxazine mononucleotide

The method of this invention requires mixing the photosensitizer with the material to be decontaminated. Mixing may be done by simply adding the photosensitizer or a solution containing the photosensitizer to a fluid to be decontaminated. In one embodiment, the material to be decontaminated to which photosensitizer has been added is flowed past a photoradiation source, and the flow of the material generally provides sufficient turbulence to distribute the photosensitizer throughout the fluid to be decontaminated. In another embodiment, the fluid and photosensitizer are placed in a photopermeable container and irradiated in batch mode, preferably while agitating the container to fully distribute the photosensitizer and expose all the fluid to the radiation.

The amount of photosensitizer to be mixed with the fluid will be an amount sufficient to adequately inactivate microorganisms therein, but less than a toxic (to humans or other mammals) or insoluble amount. As taught herein, optimal concentrations for desired photosensitizers may be readily determined by those skilled in the art without undue experimentation. Preferably the photosensitizer is used in a concentration of at least about 1 μM up to the solubility of the photosensitizer in the fluid, and preferably about 10 μM. For 7,8-dimethyl-10-ribityl isoalloxazine a concentration range between about 1 μM and about 160 μM is preferred, preferably about 10 μM.

The fluid containing the photosensitizer is exposed to UV photoradiation to activate the photosensitizer, using an amount of photoradiation sufficient to activate the photosensitizer as described above, but less than that which would cause non-specific damage to the biological components or substantially interfere with biological activity of other proteins present in the fluid. Preferably the light source is a fluorescent or luminescent source providing UV light.

The activated photosensitizer is capable of inactivating the microorganisms present, such as by interfering to prevent their replication. Specificity of action of the photosensitizer is conferred by the close proximity of the photosensitizer to the nucleic acid of the microorganism and this may result from binding of the photosensitizer to the nucleic acid. "Nucleic acid" includes ribonucleic acid (RNA) and deoxyribonucleic acid (DNA). Other photosensitizers may act by binding to cell membranes or by other mechanisms. The photosensitizer may also be targeted to the microorganism to be inactivated by covalently coupling to an antibody, preferably a specific monoclonal antibody to the microorganism.

The fluid containing the photosensitizer may be flowed into a photopermeable container for irradiation. The term "container" refers to a closed or open space, which may be made of rigid or flexible material, e.g., may be a bag or box or trough. It may be closed or open at the top and may have openings at both ends, e.g., may be a tube or tubing, to allow for flow-through of fluid therein. A cuvette has been used to exemplify one embodiment of the invention involving a flow-through system. Collection bags, such as those used with the Trima™ Spectra™ and apheresis systems of Cobe Laboratories, Inc., have been used to exemplify another embodiment involving batch-wise treatment of the fluid.

The term "photopermeable" means the material of the container is adequately transparent to photoradiation of the proper wavelength for activating the photosensitizer. In the flow-through system, the container has a depth (dimension measured in the direction of the radiation from the photoradiation source) sufficient to allow photoradiation to adequately penetrate the container to contact photosensitizer molecules at all distances from the light source and ensure inactivation

of microorganisms in the fluid to be decontaminated, and a length (dimension in the direction of fluid flow) sufficient to ensure a sufficient exposure time of the fluid to the photoradiation. The materials for making such containers, depths and lengths of containers may be easily determined by those skilled in the art without undue experimentation following the teachings hereof, and together with the flow rate of fluid through the container, the intensity of the photoradiation and the absorptivities of the fluid components, e.g., plasma, platelets, red blood cells, will determine the amount of time the fluid needs to be exposed to photoradiation. For 7,8-dimethyl-10-ribityl isoalloxazine, a preferred amount of radiation is between about $1J/cm^2$ to $120J/cm^2$.

In another embodiment involving batch-wise treatment, the fluid to be treated is placed in a photopermeable container which is agitated and exposed to photoradiation for a time sufficient to substantially inactivate the microorganisms. The photopermeable container is preferably a blood bag made of transparent or semitransparent plastic, and the agitating means is preferably a shaker table. The photosensitizer may be added to the container in powdered or liquid form and the container agitated to mix the photosensitizer with the fluid and to adequately expose all the fluid to the photoradiation to ensure inactivation of microorganisms.

Photosensitizer may be added to or flowed into the photopermeable container separately from the fluid being treated or may be added to the fluid prior to placing the fluid in the container. In one embodiment, photosensitizer is added to anticoagulant and the mixture of photosensitizer and anticoagulant are added to the fluid.

Enhancers may also be added to the fluid to make the process more efficient and selective. Such enhancers include antioxidants or other agents to prevent damage to desired fluid components or to improve the rate of inactivation of microorganisms and are exemplified by adenine, histidine, cysteine, tyrosine, tryptophan, ascorbate, N-acetyl-L-cysteine, propyl gallate, glutathione, mercaptopropionylglycine, dithiothreotol, nicotinamide, BHT, BHA, lysine, serine, methionine, glucose, mannitol, trolox, glycerol, and mixtures thereof

In decontamination systems of this invention, the photoradiation source may be connected to the photopermeable container for the fluid by means of a light guide such as a light channel or fiber optic tube which prevents scattering of the light between the source and the container for the fluid, and more importantly, prevents substantial heating of the fluid within the container. Direct exposure to the light source may raise temperatures as much as 10 to 15 ° C, especially when the amount of fluid exposed to the light is small, which can cause denaturization of blood components. Use of the light guide keeps any heating to less than about 2 ° C. The method may also include the use of temperature sensors and cooling mechanisms where necessary to keep the temperature below temperatures at which desired proteins in the fluid are damaged. Preferably, the temperature is kept between about 0°C and about 45°C, more preferably between about 4°C and about 37°C, and most preferably about 22°C.

A suitable system for treating a fluid to inactivate microorganisms which may be present therein may comprise:

(a) a container comprising said fluid and an endogenous photosensitizer or endogenously-based photosensitizer derivative, said container being equipped with input means, and having a photopermeable surface sufficient to allow exposure of the fluid therein to an amount of photoradiation sufficient to activate the photosensitizer;

(b) at least one photoradiation source for providing sufficient photoradiation to the fluid in said container of a type and amount selected to activate the photosensitizer whereby microorganisms present are substantially inactivated.

The photoradiation source is a source of ultraviolet radiation. The photoradiation source may be a simple lamp or may consist of multiple lamps radiating at differing wavelengths. The photoradiation source should be capable of delivering from about 1 to at least about 120 $J/cm^2$.

Any means for adding the photosensitizer to the fluid to be decontaminated and for placing the fluid in the photopermeable container known to the art may be used, such means typically including flow conduits, ports, reservoirs, valves, and the like. Preferably, the system includes means such as pumps or adjustable valves for controlling the flow of the photosensitizer into the fluid to be decontaminated so that its concentration may be controlled at effective levels as described above. Alternatively, the photosensitizer may be mixed with the anticoagulant feed to a blood apheresis system. For endogenous photosensitizers and derivatives having sugar moieties, the pH of the solution is preferably kept low enough, as is known to the art, to prevent detachment of the sugar moiety. Preferably the photosensitizer is added to the fluid to be decontaminated in a pre-mixed aqueous solution, e.g., in water or storage buffer solution.

The photopermeable container for the flow-through system may be a transparent cuvette made of polycarbonate, glass, quartz, polystyrene, polyvinyl chloride, polyolefin, or other transparent material. The cuvette may be enclosed in a radiation chamber having mirrored walls. A photoradiation enhancer such as a second photoradiation source or reflective surface may be placed adjacent to the cuvette to increase the amount of photoradiation contacting the fluid within the cuvette. The system preferably includes a pump for adjusting the flow rate of the fluid to desired levels to ensure substantial decontamination as described above. The cuvette has a length, coordinated with the flow rate therethrough, sufficient to expose fluid therein to sufficient photoradiation to effect substantial decontamination thereof.

Also preferably the cuvette is spaced apart from the light source a sufficient distance that heating of the fluid

in the cuvette does not occur, and light is transmitted from the light source to the cuvette by means of a light guide.

Alternatively, the fluid may be placed in a photopermeable container such as a blood bag, e.g. used with the apheresis system described in U.S. Patent No. 5,653,887, and agitated while exposing to photoradiation. Suitable bags include collection bags as described herein. Collection bags used in the Spectra™ system or Trima™ apheresis system of Cobe Laboratories, Inc. are especially suitable. Shaker tables are known to the art, e.g. as described in U.S. Patent 4,880,788. The bag is equipped with at least one port for adding fluid thereto. The photosensitizer, preferably 7,8-dimethyl-10-ribityl-isoalloxazine, may be added to the fluid-filled bag in powder form. The bag is then placed on a shaker table and agitated under photoradiation until substantially all the fluid has been exposed to the photoradiation. Alternatively, the bag may be prepackaged with the powdered photosensitizer contained therein. The fluid to be decontaminated may then be added through the appropriate port.

Decontamination systems as described above may be designed as stand-alone units or may be easily incorporated into existing apparatuses known to the art for separating or treating blood being withdrawn from or administered to a patient. For example, such blood-handling apparatuses include the COBE Spectra™ or TRIMA® apheresis systems, available from Cobe Laboratories, Inc., Lakewood, CO, or the apparatuses described in U.S. Patent 5,653,887 and U.S. Serial No. 08/924,519 filed September 5, 1997 (PCT Publication No. WO 99/11305) of Cobe Laboratories, Inc. as well as the apheresis systems of other manufacturers. The decontamination system may be inserted just downstream of the point where blood is withdrawn from a patient or donor, just prior to insertion of blood product into a patient, or at any point before or after separation of blood constituents. The photosensitizer may be added to blood components along with anticoagulant, and separate irradiation sources and cuvettes are placed downstream from collection points for platelets, for plasma and for red blood cells. The use of three separate blood decontamination systems is preferred to placement of a single blood decontamination system upstream of the blood separation vessel of an apheresis system because the lower flow rates in the separate component lines allows greater ease of irradiation.

When red blood cells are present in the fluid being treated, as will be appreciated by those skilled in the art, to compensate for absorption of light by the cells, the fluid may be thinned, exposed to higher energies of radiation for longer periods, agitated for longer periods or presented to photoradiation in shallower containers or conduits than necessary for use with other blood components.

The endogenous photosensitizers and endogenously-based derivative photosensitizers disclosed herein can be used in pre-existing blood component decontamination systems as well as in the decontamination system disclosed herein. For example, the endogenous photosensitizers and endogenously-based derivative photosensitizers of this invention can be used in the decontamination systems described in U.S. Patent Nos. 5,290,221, 5,536,238, 5,290,221 and 5,536,238.

BRIEF DESCRIPTION OF THE FIGURES

Figure 1 depicts the riboflavin absorbance spectrum.

Figure 2 depicts a correlation of light absorbance and hematocrit observed and predicted for red blood cells, and predicted for platelets.

Figure 3 depicts photodecomposition over time of riboflavin in anticoagulant Acid Citrate Dextrose (ACD) solution. The solid line with circles indicates percent of initial riboflavin remaining at 373 nm. The dotted line with squares indicates percent of initial riboflavin remaining at 447 nm.

Figure 4 depicts the transmission profile of various plastic cuvettes as a function of wavelength. The solid line represent a 3.2 mm acrylic cuvette. The dotted line (-----) represents a 3.2 mm UV acrylic cuvette. The dashed line (——) represents a 3.2 mm polystyrene (PS) cuvette, and the crossed line indicates a 3.2 mm polycarbonate (PC) cuvette.

Figure 5 depicts the light flux required in mW per $cm^2$ as a function of flow rate, i.e. the flux required to deliver one joule/$cm^2$ to a sample in the cuvette.

Figure 6 depicts a blood separation apparatus incorporating the photoradiation device of this invention.

Figure 7 depicts the decontamination assembly of this invention.

Figure 8 depicts inactivation of bacteria in platelet preparations using vitamin K5 as the photosensitizer as a function

of energy of irradiation.

Figure 9 depicts inactivation of bacteria as a function of platelet preparation and energy of irradiation, using 90% platelets and 10% platelet additive solution (90:10) and 30% platelets with 70% additive solution (30:70).

Figure 10 shows the effect on inactivation of virus, bacteriophage and bacteria of adding antioxidants to platelet concentrate.

Figure 11 shows the inactivation curve for Herpes Simplex type II virus as a function of concentration of photosensitizer at an energy of irradiation of $20J/cm^2$ using half ultraviolet and half visible light.

Figure 12 shows inactivation of *S. epidermidis* at varying concentrations of photosensitizer and energies of irradiation.

Figure 13 shows inactivation of ΦX174 at varying concentrations of photosensitizer and energies of irradiation.

Figure 14 shows inactivation of *S. aureus* and ΦX174 at varying energies of irradiation using a 50:50 mixture of ultraviolet and visible light.

Figure 15 shows inactivation of *S. epidermidis* and HSV-II at varying energies of irradiation using a 50:50 mixture of ultraviolet and visible light.

Figure 16 shows inactivation of HSV2 virus in blood bags agitated and irradiated at varying energy levels.

Figure 17 compares inactivation results for vaccinia virus in various fluids using ultraviolet light alone or 50:50 visible and ultraviolet light.

Figure 18 compares inactivation results with and without sensitizer of vaccinia virus at varying irradiation times.

Figure 19 compares inactivation of extracellular HIV-1 at 5 and 50 μM of photosensitizer and varying irradiation energies.

Figure 20 compares inactivation of intracellular HIV-1 at 5 and 50 μM of photosensitizer and varying irradiation energies.

Figure 21 compares inactivation of intracellular HIV-1 at 5 and 50 μM of photosensitizer and varying irradiation energies, using p24 antigen levels.

Figure 22 shows inactivation of HSV-II at varying irradiation levels using platelet concentrate and platelet concentrate in media containing platelet additive solution with ascorbate.

Figure 23 shows an embodiment of this invention using a blood bag to contain the fluid being treated and photosensitizer and a shaker table to agitate the fluid while exposing to photoradiation from a light source.

## DETAILED DESCRIPTION

The decontamination method of this invention using endogenous alloxazine photosensitizers and endogenously-based alloxazine derivative photosensitizers is exemplified herein using 7,8-dimethyl-10-ribityl isoalloxazine as the photosensitizer, however, any photosensitizer may be used which is capable of being activated by photoradiation to cause inactivation of microorganisms. The photosensitizer must be one which does not destroy desired components of the fluid being decontaminated, and also preferably which does not break down as a result of the photoradiation into products which significantly destroy desired components or have significant toxicity. The wavelength at which the photosensitizer is activated is determined as described herein, using literature sources or direct measurement Its solubility in the fluid to be decontaminated or in a combination of carrier fluid and fluid to be contaminated is also so determined. The ability of photoradiation at the activating wavelength to penetrate the fluid to be decontaminated must also be determined as taught herein. Appropriate temperatures for the reaction of the photosensitizer with its substrate are determined, as well as the ranges of temperature, photoradiation intensity and duration, and photosensitizer concentration which will optimize microbial inactivation and minimize damage to desired proteins and/or cellular components in the fluid. Examples 1-7 and Figures 1-5 illustrate the determination of information required to develop a flow-through decontamination system.

Once such system requirements have been determined for flow-through systems, apparatuses may be designed which provide the correct flow rates, photopermeabilities, and light intensities to cause inactivation of microorganisms present in the fluid, as is taught herein. The fluid to be decontaminated is mixed with photosensitizer and then irradiated with a sufficient amount of photoradiation to activate the photosensitizer to react with microorganisms in the fluid such that microorganisms in the fluid are inactivated. The amount of photoradiation reaching microorganisms in the fluid is controlled by selecting an appropriate photoradiation source, an appropriate distance of the photoradiation source from the fluid to be decontaminated, which may be increased through the use of light guides to carry the photoradiation directly to the container for the fluid, an appropriate photopermeable material for the container for the fluid, an appropriate depth to allow full penetration of the photoradiation into the container, photoradiation enhancers such as one or more additional photoradiation sources, preferably on the opposite side of the container from the first, or reflectors to reflect light from the radiation source back into the container, appropriate flow rates for the fluid in the container and an appropriate container length to allow sufficient time for inactivation of microorganisms present. Temperature monitors and controllers may also be required to keep the fluid at optimal temperature. Figure 6 depicts a decontamination system useful for carrying out the method of this invention as part of an apparatus for separating blood components, and Figure 7 provides details of a preferred decontamination system.

For batch systems, it is preferred to place the fluid to be decontaminated along with photosensitizer in bags which are photopermeable or at least sufficiently photopermeable to allow sufficient radiation to reach their contents to activate the photosensitizer. Sufficient photosensitizer is added to each bag to provide inactivation, preferably to provide a photosensitizer concentration of at least about 10 μM, and the bag is agitated while irradiating, preferably at about 1 to about 120 J/cm$^2$ for a period of between about 6 and about 36 minutes to ensure exposure of substantially all the fluid to radiation. The photosensitizer may be added in powdered form.

The method uses endogenous alloxazine photosensitizers, 7,8-dimethyl-10-ribityl isoalloxazine is the preferred photosensitizer for use in this invention. The chemistry believed to occur between 7,8-dimethyl-10-ribityl isoalloxazine and nucleic acids does not proceed via singlet oxygen-dependent processes (i.e. Type U mechanism), but rather by direct sensitizer-substrate interactions (Type I mechanisms). Cadet et al. (1983) J. Chem., 23:420-429, clearly demonstrate the effects of 7,8-dimethyl-10-ribityl isoalloxazine are due to non-singlet oxygen oxidation of guanosine residues. In addition, adenosine bases appear to be sensitive to the effects of 7,8-dimethyl-10-ribityl isoalloxazine plus UV light. This is important since adenosine residues are relatively insensitive to singlet oxygen-dependent processes. 7,8-dimethyl-10-ribityl isoalloxazine appears not to produce large quantities of singlet oxygen upon exposure to UV light, but rather exerts its effects through direct interactions with substrate (e.g., nucleic acids) through electron transfer reactions with excited state sensitizer species. Since indiscriminate damage to cells and proteins arises primarily from singlet oxygen sources, this mechanistic pathway for the action of 7,8-dimethyl-10-ribityl isoalloxazine allows greater selectivity in its action than is the case with compounds such as psoralens which possess significant Type II chemistry.

Figure 6 shows a blood apparatus device and apheresis system incorporating the photoradiation devices of this invention. Whole blood is withdrawn from a donor/patient **4** and is provided to an apheresis system or blood component separation device **8** where the blood is separated into the various component types and at least one of these blood component types is removed from the device **8.** These blood components may then be provided for subsequent use by another or may undergo a therapeutic treatment and be returned to the donor/patient **4.**

In the blood component separation device **8,** blood is withdrawn from the donor/patient 4 and directed through an extracorporeal tubing circuit **10** and a blood-processing vessel **12,** defining a completely closed and sterile system. The blood component separation device **8** is connected to a pump (not shown). Blood flows from the donor/patient **4** through the extracorporeal tubing circuit **10** and into rotating blood processing vessel **12.** The blood within the blood processing vessel **12** is separated into various blood component types, and these component types (platelets, plasma, red blood cells) are continually removed from the blood processing vessel **12.** Blood components which are not being retained for collection or for therapeutic treatment (e.g., red blood cells, white blood cells, plasma) are also removed from the blood processing vessel **12** and returned to the donor/patient **4** via the extracorporeal tubing circuit **10.**

Operation of the blood component separation device is preferably controlled by one or more computer processors included therein.

Extracorporeal tubing circuit **10** comprises a cassette assembly **14** and a number of tubing assemblies **20, 50, 60, 80, 90, 100** interconnected therewith. Blood removal/return tubing assembly **20** provides a single needle interface between a donor/patient 4 and cassette assembly **14,** and blood inlet/blood component tubing subassembly **60** provides the interface between cassette assembly **14** and blood processing vessel **12.** An anticoagulant tubing assembly **50,** platelet collection tubing assembly **80,** plasma collection tubing assembly 90, red blood cell collection tubing assembly **70** and vent bag tubing subassembly 100 are also interconnected with cassette assembly **14.**

The blood removal/return tubing assembly **20** includes a needle subassembly **30** interconnected therewith and anticoagulant tubing **26** connecting to anticoagulant tubing assembly **50** through cassette assembly **14.**

Cassette assembly **14** includes front and back molded plastic plates that are hot-welded together to define a

rectangular cassette member having integral fluid passageways. The cassette assembly **14** further includes a number of outwardly extending tubing loops interconnecting various integral passageways. The integral passageways are also interconnected to the various tubing assemblies.

Specifically, cassette assembly **14** interconnects with anticoagulant tubing **26** of the blood removal/return tubing assembly **20** and with anticoagulant tubing assembly **50.** The anticoagulant tubing assembly **50** includes a spike drip chamber **52** connectable to anticoagulant and photosensitizer source **53** and a sterilizing filter **56.** During use, the anticoagulant tubing assembly **50** supplies anticoagulant mixed with photosensitizer to the blood removed from donor/patient **4** to reduce or prevent any clotting in the extracorporeal tubing circuit **10.** Many anticoagulant are known to the art, e.g. as disclosed in Chapter 3 of the AABB Technical Manual, 11th edition, 1993, including ACD-A, CPD, CP2D, CPDA-1 and heparin. These as well as cell storage solutions , AS-1, AS-3 and AS-5, are all compatible with the endogenous photosensitizers and endogenously-based derivative photosensitizers described herein.

Cassette assembly **14** also includes an interconnection with blood removal tubing of the blood removal/return tubing assembly **20.** Blood passes through pressure sensors, and an inlet filter in cassette assembly **14** and thence to blood inlet tubing **62.** Blood inlet tubing **62** is also interconnected with blood processing vessel **12** to provide whole blood thereto for processing.

To return separated blood components to cassette assembly **14,** the blood inlet/blood component tubing assembly **60** further includes red blood cell (RBC)/plasma outlet tubing, platelet outlet tubing and plasma outlet tubing interconnected with corresponding outlet ports on blood processing vessel **12.** The red blood cell (RBC)/plasma outlet tubing channels the separated red blood cell (RBC)/plasma component through cassette assembly **14** to red blood cell collection tubing assembly **70** through first decontamination system **72.** The platelet outlet tubing channels separated platelets through cassette assembly **14** to platelet collection tubing assembly **80** through second decontamination system **82.** The plasma outlet tubing channels separated plasma through cassette assembly **14** to plasma collection tubing assembly **90** through third decontamination system **92.** After irradiation in the decontamination systems **72, 82** and **92,** to activate the photosensitizer and inactivate microorganisms present, the blood components are collected in red blood cell collection bag **74,** platelet collection bags **84,** and plasma collection bag **94.** Vent bag **104** may be used to vent gases within the system.

Figure 7 depicts a stand-alone version of the decontamination assembly of this invention. Blood product **180** (which may be recently collected blood or blood component or stored blood) is connected to blood product line **186** which leads through pump **184** to decontamination cuvette **164.** Photosensitizer reservoir **166** is connected to photosensitizer input line **168** equipped with input pump **170,** and leads into blood product line **186** upstream from decontamination cuvette **164.** Decontamination cuvette **164** is a photopermeable cuvette of a depth (d) and a length (1) selected to ensure decontamination. Cooling system **190** combined with temperature monitor **192** are connected with decontamination cuvette **164** for controlling the temperature of the fluid. Decontamination cuvette **164** is connected via light guide **162** to photoradiation source **160.** A photoradiation enhancer **163** is placed adjacent to (either touching or spaced apart from) decontamination cuvette **164** to increase the amount of photoradiation reaching the blood product in the cuvette. Decontaminated blood product line **188** leads from decontamination cuvette **164** to decontaminated blood product collection **182.**

In operation, blood product **180** is conducted into blood product line **186** where it is joined by photosensitizer from photosensitizer reservoir **166** flowing at a rate controlled by photosensitizer input pump **170** in photosensitizer input line **68** which joins blood product line **186.** The flow rate in blood product line **186** is controlled by pump **184** to a rate selected to ensure decontamination in decontamination cuvette **164.** Temperature monitor **192** measures the temperature of fluid in cuvette **164** and controls cooling system **190** which keeps the temperature in the cuvette within a range required for optimal operation. The blood product in decontamination cuvette **164** is irradiated by photoradiation from photoradiation source **160** conducted in light guide **162.** The photoradiation source may comprise two or more actual lights. The arrows indicate photoradiation from the end of light guide **162** propagating in the blood product inside transparent decontamination cuvette **164.** Adjacent to decontamination cuvette **164** is photoradiation enhancer **163** which may be an additional source of photoradiation or a reflective surface. The arrows from photoradiation enhancer **163** pointing toward decontamination cuvette **164** indicate photoradiation from photoradiation enhancer **163** shining on the blood product material in cuvette **164.** Decontaminated blood product exits decontamination cuvette **164** via decontaminated blood product line **188** and is collected at decontaminated blood product collection **182.**

In one embodiment using 7,8-dimethyl-10-ribityl isoalloxazine from Sigma Chemical Company as the photosensitizer, a light guide from EFOS Corporation, Williamsville, N.Y. composed of optical fibers is used. The system is capable of delivering a focused light beam with an intensity of 6,200 mW/cm$^2$ in the region of 355-380 nm. It is also possible to use interchangeable filters with the system to achieve outputs of 4,700 mW/cm$^2$ in the spectral region of 400-500 nm. In both cases, the output of light in the region of 320 nm and lower is negligible. Light guides of varying dimensions (3, 5 and 8 mm) are available with this system. The light exits the light guide tip with a 21 degree spread. The 8 mm light guide is appropriate, correctly placed, to adequately illuminate the face of the preferred decontamination cuvette which is a standard cuvette used on Cobe Spectra® disposables sets from Industrial Plastics, Inc., Forest Grove,

OR.

The flow rate is variable and is determined by the amount of light energy intended to be delivered to the sample. The flow rate is controlled by means of a peristaltic pump from the Cole-Parmer Instrument Company, Vernon Hills, IL. Flow rates and type of input stream may be controlled via a computer processor as is known to the art.

Figure 23 depicts an embodiment of this invention in which fluid to be decontaminated is placed in a blood bag **284** equipped with an inlet port **282,** through which photosensitizer in powder form **284** is added from flask **286** via pour spout **288.** Shaker table **280** is activated to agitate the bag **284** to dissolve photosensitizer **290** while photoradiation source **260** is activated to irradiate the fluid and photosensitizer in bag **284.** Alternatively, the bag can be provided prepackaged to contain photosensitizer and the fluid is thereafter added to the bag.

The methods of this invention do not require the use of enhancers such as "quenchers" or oxygen scavengers, however these may be used to enhance the process by reducing the extent of non-specific cell or protein-damaging chemistry or enhancing the rate of pathogen inactivation. Further preferred methods using non-toxic endogenous photosensitizers and endogenously-based derivative photosensitizers do not require removal of photosensitizers from the fluid after photoradiation. Test results show little or no damage to other blood components, e.g. platelets remain biologically active five days post-treatment.

EXAMPLES

## Example 1. Absorbance Profile of 7,8-dimethyl-10-ribityl isoalloxazine

A sample of 7,8-dimethyl-10-ribityl isoalloxazine (98% purity) was obtained from Sigma Chemical Company. A portion of this sample was submitted for analysis using a scanning UV spectrophotometer. The range studied covered the region of 200 to 900 nm. For analysis, the sample was dissolved in distilled water. A sample spectrum from this analysis is shown in Figure 1.

Results were consistent with those reported in the literature for the absorbance maxima and extinction coefficients for 7,8-dimethyl-10-ribityl isoalloxazine

| Literature λmax (ε) | Measured λmax (ε) |
|---|---|
| 267 (32,359) | 222 (30,965)<br>265 (33,159) |
| 373 (10,471) | 373 (10,568) |
| 447 (12,303) | 445 (12,466) |

Appropriate wavelengths for irradiation are 373 and 445 nm. The extinction coefficients observed at these absorbance maxima is sufficient to ensure adequate activation of the sensitizer in solution.

## Example 2. Solubility of 7,8-dimethyl-10-ribityl isoalloxazine

Solubility in Isolyte S, pH 7.4 Media

The maximum solubility of 7,8-dimethyl-10-ribityl isoalloxazine in Isolyte S media was determined as follows:

7,8-dimethyl-10-ribityl isoalloxazine was mixed with Isolyte S until a precipitate was formed. The mixture was agitated at room temperature for one hour and vortex mixed to ensure complete dissolution of the suspended material. Additional 7,8-dimethyl-10-ribityl isoalloxazine was added until a solid suspension remained despite additional vortex mixing. This suspension was then centrifuged to remove undissolved material. The supernatant from this preparation was removed and analyzed using a spectrophotometer. The absorbance values of the solution were determined at 447 nm and 373 nm. From the extinction coefficients that were determined previously, it was possible to estimate the concentration of the saturated solution

$$\text{Concentration (373)} = 110\ \mu M = 42\ \mu g/mL$$

$$\text{Concentration (447)} = 109\ \mu\text{M} = 40.9\ \mu\text{g/mL}$$

Solubility in ACD-A Anticoagulant

The same procedure described above was repeated using ACD-A Anticoagulant. The values obtained from these measurements were as follows:

$$\text{Concentration (373)} = 166\ \mu\text{M} = 63\ \mu\text{g/mL}$$

$$\text{Concentration (447)} = 160\ \mu\text{M} = 60.3\ \mu\text{g/mL}$$

The values obtained from these studies indicate an upper limit of solubility of the compound that may be expected.

**Example 3. Photodecomposition of 7,8-dimethyl-10-ribityl isoalloxazine in Aqueous Media**

A solution of 7,8-dimethyl-10-ribityl isoalloxazine in Sigma ACD-A was prepared at a concentration of 63 μg/mL. This preparation was taken up into a glass pipette and placed in the path of a UV light source (365 nm λmax with filters to remove light below 320 nm). The suspension was irradiated for specific intervals at which aliquots were removed for spectroscopic analysis. The absorbance of the dissolved 7,8-dimethyl-10-ribityl isoalloxazine was monitored at 373 and 447 nm at each time interval. The results are depicted in Figure 3 and Table 1.

Table 1. Photodecomposition of 7,8-dimethyl-10-ribityl isoalloxazine Upon Exposure to UV Light (365 nm) in Acid Solution

| Irradiation Time | % of Initial, 373 nm | % of Initial, 447 nm |
|---|---|---|
| 0 | 100 | 100 |
| 5 | 87.3 | 61.6 |
| 10 | 90.5 | 76.6 |
| 15 | 100 | 70 |

The absorption profile for the solution at 373 nm indicates that no significant decomposition of the reagent occurred over the entire irradiation period. The absorbance of light at this wavelength corresponds to n-π* electronic transitions. The absence of a decrease in the intensity of this peak over time indicates that the ring structure of the molecule is intact despite prolonged irradiation under these conditions. The absorbance of the molecule at 447 nm is due to π-π* electronic state transitions. The decrease in the absorbance of the molecule at this wavelength with increasing irradiation times is indicative of subtle alterations in the resonance structure of the molecule. This change is most likely due to the loss of ribose from the ring structure of the 7,8-dimethyl isoalloxazine backbone and the formation of 7,8-dimethylalloxozine as a result. These changes are consistent with literature reports on the behavior of the molecule upon irradiation with UV light.

The apparent lack of decomposition of the ring structure of the molecule is in stark contrast to observations with psoralen based compounds under similar conditions. During irradiation, a significant fluorescence of the molecule in solution was observed. This behavior of the molecule is consistent with the resonance features of the ring structure and provides a means for the dissipation of energy in the excited state molecule in a non-destructive fashion.

**Example 4. Flow System Concept Evaluation**

Light Transmission Properties of Existing Spectra Cuvette

The existing Spectra cuvette is composed of polycarbonate. The light transmission properties of this cuvette

were measured at 373 and 447 nm by placing the cuvette in the light path of a UV spectrophotometer. The values obtained were as follows:

| Wavelength of Light | % Transmittance |
|---|---|
| 373 nm | 66% |
| 447 nm | 80% |

These results are consistent with those reported in the literature for polycarbonate plastics (see Figure 4). The literature values indicate a steep shoulder for the transmission of light through polycarbonates in the region of 300 nm. For the region above 350 nm, the light transmission properties are adequate for this application.

Light Flux Requirements Calculated as a Function of Flow Rates

In order for a flow system to be feasible, the sample must be provided with an adequate flux of light during its presence in the beam path. If the proposed Spectra cuvette were to serve this purpose, then it is possible to estimate the light flux requirements as a function of flow rates through the cuvette as follows:

The volume of solution present in the irradiation zone of the cuvette is ca. 0.375 mls.
The transit time for a cell in this region of the cuvette can be determined from the following equation:

$$T = \frac{\text{Volume of Cuvette (mls)}}{\text{Flow Rate (mls/min)}}$$

At 100 mls per minute, the transit time (T) would be 0.00375 min = 0.225 seconds.

The energy to which a sample is exposed is dependent on the flux according to the following equation:

$$\text{Energy (E, Joules/cm}^2) = \frac{\text{Flux } (\phi, \text{mW/cm}^2) * \text{Time (T, sec.)}}{1000}$$

If we assume that 1 Joule/cm$^2$ is required to activate the sensitizer adequately and the transit time (T) is 0.22 seconds (i.e., flow rate of 100 mls/min through the cuvette), then the required Flux during the sample's transit through the cuvette is 4,545 mW/cm$^2$. A graph depicting the relationship of the required flux from the light source to flow rates through the cuvette is provided in Figure 5.

These results indicate that, for a flow system to operate properly, UV sources with outputs in the region of Watts/cm$^2$ are required.

Figure 2 shows how absorbance should vary with concentration of platelets.

**Example 5. Absorbance of Red Blood Cells.**

In order to evaluate the extent to which UV light can penetrate a red cell sample and the effects of sample thickness and hematocrit on the extent of light penetration, several preliminary experiments were carried out using chemical actinometry, a method for determining the actual amount of light intensity emanating from a source by measuring the ability and extent to which absorbed light can effect a chemical reaction. For these studies, a ferrioxalate solution was utilized in order to measure the source intensity relative to that observed for water. Details of the chemical reaction and the methods utilized for sample preparation are as taught in Gordon, A.J. and Ford, R.A. (1972), "The Chemist's Companion: A Handbook of Practical Data, Techniques and References" (John Wiley & Sons), pp. 362-368.

Samples of iron (III) oxalate were prepared in the test material (water or blood product at varying red cell hematocrits) at a concentration of 0.15 M. These samples were then loaded into a standard Spectra cuvette and placed in the irradiation assembly. Samples were exposed for pre-determined time intervals corresponding to the desired energy dose level (1 J/cm$^2$). The samples were then removed and the amount of conversion of $Fe^{3+}$ to $Fe^{2+}$ was determined by reading the absorbance of the test article in a 1,10-phenanthroline solution at 510 nm as described in Gordon, A.J. and Ford, R.A., *supra.* Higher absorbance values are indicative of greater light penetration into the sample. The absorbance value observed for water after exposure to 1 J/cm$^2$ UV radiation was used as the 100% Transmittance level. All

values for red cell samples were determined relative to this standard.

Table 2. Absorbance Readings After Exposure of Samples to 1 J/cm$^2$ UVA Light. All Average Values Represent the Mean of 6 Experiments. % Transmittance Values Are Calculated Relative to Water Samples.

| Absorbance at 510nm | Average | Standard Deviation | % Transmittance | Standard Deviation |
|---|---|---|---|---|
| Water | 2.40 | 0.04 | 100 | 0.0 |
| RBC, 1.3% Hematocrit | 2.40 | 0.10 | 99.5 | 4.8 |
| RBC, 3.7% Hematocrit | 1.46 | 0.38 | 60.6 | 15.4 |
| RBC, 5.07% Hematocrit | 0.20 | 0.26 | 8.3 | 10.8 |
| RBC, 6.0% Hematocrit | 0.13 | 0.09 | 5.2 | 3.9 |
| RBC, 10.2% Hematocrit | 0.23 | 0.19 | 9.7 | 7.9 |
| RBC, 16.3% Hematocrit | 0.25 | 0.11 | 10.4 | 4.6 |
| RBC, 21.8% Hematocrit | 0.09 | 0.06 | 3.6 | 2.6 |
| RBC, 80.2% Hematocrit | 0.01 | 0.11 | 0.3 | 4.4 |

Using these values, it is possible to calculate the penetration depth of UV light by using Beer's Law (A = ∈ b C). From Lambert's Law,

$$\text{Absorbance} = \text{Log}\ (1/\text{Transmittance})$$

If we let the concentration (C) be equal to the hematocrit of the sample, and since b = 0.3 cm (the path length of the Spectra cuvette), then it is possible to determine a pseudo-extinction coefficient for the samples (∈') by plotting the absorbance values for the red cell samples versus the product of the hematocrit times the path length. The extinction coefficient for the samples is represented by the slope of this line.

Table 3: Determination of Extinction Coefficient for Red Cell Samples.

| T | B | HCT | B*HCT | Absorbance log (1/T) | ∈ |
|---|---|---|---|---|---|
| 0.995 | 0.3 | 1.3 | 0.39 | 0.002 | .0051 |
| 0.606 | 0.3 | 3.7 | 1.11 | 0.218 | .196 |
| 0.0525 | 0.3 | 6 | 1.8 | 1.280 | .71 |
| 0.097 | 0.3 | 10.2 | 3.06 | 1.013 | .33 |
| 0.104 | 0.3 | 16.3 | 4.89 | 0.983 | .20 |
| 0.036 | 0.3 | 21.8 | 6.54 | 1.444 | .22 |
| 0.0033 | 0.3 | 80.2 | 24.06 | 2.481 | .10 |

Using the values obtained as described above, it was possible to determine a pseudo-extinction coefficient for these samples to be 0.08661.

The value for the extinction coefficient permits calculation of the penetration distance of UV light into red cell

samples as a function of the sample hematocrit. For this estimation, the penetration depth of the sample in which 90% of the incident light would be absorbed was determined using the following equation:

$$A = \epsilon\ b\ C$$

A = 1 (90% Absorbance of Incident Light), $\epsilon$ = 0.08661, C = Sample hematocrit, b = Path Length.

The values determined using actinometry were compared to those which were calculated previously using estimates taken from UV Spectrophotometric measurements of light absorbance in red cell and platelet samples.

Figure 2 shows how absorbance and distance from the light source varies for red blood cells, comparing predicted with observed values. These results indicate that, for samples at hematocrits in the region of 80%, it is possible, using the preferred configuration of this invention, to get light into the sample to a depth of 0.14 cm. This represents a flow path width that is less than half the width of the current Spectra cuvette.

**Example 6. Effects of Virus Inactivation Treatment on Platelet *In Vitro* Parameters.**

Effects of virus inactivation treatment on platelet *in vitro* parameters were evaluated. Platelet preparations were treated with 7,8-dimethyl-10-ribityl isoalloxazine in combination with UV light. Various *in vitro* parameters were used as monitors of platelet function in order to determine the extent of changes induced by the treatment conditions. Factors such as energy level of UV light exposure, dose of 7,8-dimethyl-10-ribityl isoalloxazine used, and sample processing conditions were examined for their impact on platelet quality post-treatment. Results from this study are used to establish an appropriate treatment window for inactivation of HIV-1 without compromising platelet function.

Samples were prepared with three different concentrations of 7,8-dimethyl-10-ribityl isoalloxazine. Platelets obtained from a standard Spectra LRS collection were used for these studies.

Starting samples were centrifuged to concentrate the platelet pellet. The pellet was resuspended in a 70:30 (Isolyte S, pH 7.4; McGaw, Inc. Media:Plasma) solution. 7,8-dimethyl-10-ribityl isoalloxazine at the specified concentration, was present in the plasma:media mixture. The platelet suspension was then passed through a UV irradiation chamber at one of three specified flow rates. The flow rates were directly correlated to the energy level of exposure for the cells/media mixture which passes through the irradiation chamber. After flowing through the irradiation chamber, samples were stored in a citrate plasticized sampler bag for subsequent analysis.

Following irradiation, *in vitro* measurements of platelet function, including hypotonic shock response (HSR), GMP-140 expression, pH, $pCO_2$, $pO_2$, platelet swirl, and cell count, were evaluated in order to determine the effects of the treatment protocol on cell quality.

Platelet quality was monitored as a function of irradiation conditions (sensitizer concentration and flow rates/ Energy levels). The platelet quality includes parameters such as HSR response, GMP-140 activation, etc. The flow rates that are studied can be related to the Energy of exposure as follows:

$$\text{Transit Time (T, sec)} = \text{Exposure Time} = \frac{0.375\ \text{mls}}{(F_r/60)}$$

$F_r$ = Flow Rate (mls/min)
0.375 mls = Cuvette Volume (mls)

$$\therefore\ T\ (\text{sec}) = \frac{22}{F_r}$$

$$\text{Energy (Joules/cm}^2) = \frac{\text{Flux } (\phi,\ \text{mW/cm}^2) * T\ (\text{sec})}{1000}$$

$$E = \frac{\phi * 0.022}{F_r}$$

The effect of energy of UV exposure and concentration of 7,8-dimethyl-10-ribityl isoalloxazine on the stability and viability of treated platelets was evaluated. Three energy levels and three concentration levels were evaluated as follows:

| | |
|---|---|
| Energy Levels: | 1,5,9 $J/cm^{2*}$ |
| 7,8-dimethyl-10-ribityl isoalloxazine Concentrations: | 1, 50, 100 μM** |

* Levels of total energy exposure were determined by the flow rate of the suspension through the irradiation chamber in accordance with the conversion chart of Table 4.
** Since the media is diluted 70:30 (Media:Plasma) the stock concentration of 7,8-dimethyl-10-ribityl isoalloxazine in media alone prior to mixing with the plasma was adjusted appropriately. This required starting concentrations in Isolyte S of 1.43, 71.4, and 143 μM.

Table 4. Energy Exposure Levels as a Function of Flow Rate Through the Irradiation Chamber

| Energy Delivered ($J/cm^2$) | Flow Rate (mls/min) | Time to process 20 mls (minutes) |
|---|---|---|
| 1 | 16.90 | 1.18 |
| 2 | 8.45 | 2.37 |
| 3 | 5.83 | 3.55 |
| 4 | 4.22 | 4.73 |
| 5 | 3.38 | 5.92 |
| 6 | 2.82 | 7.10 |
| 7 | 2.41 | 8.29 |
| 8 | 2.11 | 9.47 |
| 9 | 1.88 | 10.65 |
| 10 | 1.69 | 11.84 |
| Flux = 3640 $mW/cm^2$; chamber volume = 0.117 mls. | | |

Values for treated samples were compared to control groups. The control samples included the following:

Untreated Sample in Plasma (Historical Control)
+Flow-UV-7,8-dimethyl-10-ribityl isoalloxazine

Procedure

A normal donor platelet apheresis product was obtained from an AABB accredited blood banking facility. The sample was collected using standard Spectra LRS procedures. All manipulations or procedures described below were performed with standard laboratory safety procedures and methods. The unit number and blood type were recorded. All samples were used within 24 hours of collection. Aseptic procedure was followed for all sample transfers and processing steps.

The sample was transferred to a 500 mls PVC transfer pack and centrifuged at 5000 x g for five minutes to pack the platelets. Plasma was then removed from the platelet pellet using a standard plasma press. The plasma was retained for further use. The plasma removed from the cell pellet was then mixed with a stock solution of Isolyte S, pH 7.4; McGaw, Inc. This stock solution of media was prepared by adding a pre-determined amount of 7,8-dimethyl-10-ribityl isoalloxazine to Isolyte S to provide final concentrations of 1.43, 71.4, and 143 μM. Following addition of 7,8-dimethyl-10-ribityl isoalloxazine the stock solution was filtered through a 0.22 μM sterile filter. The stock solution was then mixed with autologous plasma in a 70:30 (v:v) ratio to provide final 7,8-dimethyl-10-ribityl isoalloxazine concentrations of 1, 50, and 100 μM respectively. During preparation of the 7,8-dimethyl-10-ribityl isoalloxazine stock solutions, care was taken to avoid exposure to light. Samples were prepared according as follows:

| | |
|---|---|
| 1 μM | 2 samples |
| 100 μM | 2 samples |
| 50 μM | 1 sample |

The platelet pellet was then resuspended in the plasma:media mixture to the original volume of the starting sample. The sample was connected to a flow apparatus comprising a container for cells and photosensitizer, a container for media, said containers being connected via valved lines to a single line for mixed cells/sensitizer and media equipped with a pump. Mixed cells/sensitizer and media were flowed into a cuvette held in a holder with a mirrored wall, irradiated by a light source. This irradiation chamber was equipped with a temperature probe. After passing through the cuvette, fluid was collected in a product bag.

The tubing set was initially primed with Isolyte S media. Five minutes prior to the start of the test sample flow, the light source was activated. Temperature was monitored during this interval and kept lower than 32°C in the irradiation chamber.

The flow rate for the sample through the irradiation chamber was determined by the chart of Table 4. Flow rates which provide total irradiation energy levels of 1, 5 and 9 J/cm$^2$ were utilized according to the following testing matrix:

**Sample Run #1: 7,8-dimethyl-10-ribityl isoalloxazine Concentration = 1 μM**

A. +7,8-dimethyl-10-ribityl isoalloxazine+ 1 J/cm$^2$
B. +7,8-dimethyl-10-ribityl isoalloxazine+ 9 J/cm$^2$

**Sample Run #2: 7,8-dimethyl-10-ribityl isoalloxazine= 100 μM**

A. + 7,8-dimethyl-10-ribityl isoalloxazine+ 1 J/cm$^2$
B. + 7,8-dimethyl-10-ribityl isoalloxazine+ 9 J/cm$^2$

**Sample Run #3: 7,8-dimethyl-10-ribityl isoalloxazine= 50 μM**

A. + 7,8-dimethyl-10-ribityl isoalloxazine+ 5 J/cm$^2$

**Sample Run #4: Control Sample, 7,8-dimethyl-10-ribityl isoalloxazine = 0 μM**

A. +Flow-UV-7,8-dimethyl-10-ribityl isoalloxazine

All samples were identified by the run number and sample letter designation corresponding to treatment condition (i.e., 1A). Each sample set was run for a total of 2 replicates. The order in which samples were treated was determined by assignment according to a random number generator.

A sample volume of 20 mls per run condition was collected for each sample. These samples were collected into citrate plasticized sampling bags (53 mls total volume) and stored for analysis. The temperature of the sample and the irradiation chamber was noted at the start, mid-point, and end of each run.

An initial aliquot from each preparation was removed post-treatment for analysis. Parameters for analysis included cell count, pH, $pCO_2$, $pO_2$, platelet swirl, HSR, and GMP-140 analysis. The remaining portion of the sample was placed in an end-over-end platelet agitator in a +22 incubator and stored for five days post-treatment. On day 5, a second aliquot was removed and analyzed for the same *in vitro* parameters.

The following equipment was used: Nikon Labophot microscope; Serono-Baker System 9000 Hematology Analyzer; analytical balance; platelet incubator (+22 Celsius) and rotator; laboratory refrigerator (+4 Celsius); Mistral 3000i Centrifuge; Corning Blood Gas Analyzer; Becton-Dickinson FACSCALIBUR Flow Cytometer; UV irradiation chamber; UV radiometer (UVX Radiometer, UVP, Inc.); EFOS Ultracure 100SS Plus (365 nm maximum output and 340 nm bandpass filters); and temperature probe (thermocouple).

Results for each set of test variables were compared for the defined conditions of energy of exposure and concentration of 7,8-dimethyl-10-ribityl isoalloxazine. Direct comparison to the untreated control sample was made and significant differences defined by a probability $p>0.05$ from a paired, one-tailed, Student's T-Test analysis.

The results from these studies were summarized as follows:

1. At sensitizer concentrations in excess of 10 μM and platelet concentrations above 1.5E+06/μL, there was a drop in sample pH by day 2. The pH declined steadily beyond day 2 of storage reaching unacceptable levels (<6.5) by day 3 of storage. All other *in vitro* parameters followed the pattern observed with sample pH.

2. This decrease in sample pH occurred regardless of whether or not the sample was exposed to UV light.

3. At platelet concentrations of 5.4E+05/μL, there was no drop in sample pH after extended storage at any sensitizer concentration studied up to 100 μM.

4. At sensitizer concentrations up to 10 μM, platelet concentrations above 1.5E+06/μL, and UVA levels up to 10 J/$cm^2$, measured platelet properties were comparable to control, untreated cells. These remained comparable to control levels after five or more days of storage post-treatment.

These studies on platelet function post-treatment provided a clear window in which cell properties were maintained at levels comparable to untreated cells. The results also indicated that by varying the storage or treatment conditions for the cells this window can be expanded. The observed effect of 7,8-dimethyl-10-ribityl isoalloxazine with or without UV light on sample pH suggests a metabolic effect of this additive which may be moderated by changes in the storage or processing conditions of the samples.

**Example 7. Measurements of Shear Stresses on Red Cells As a Function of Flow Rate and Sample Hematocrit**

The low levels of UV light penetration into red cell samples at high hematocrits raised the need to understand the effects of passing red cells through narrow openings in the light path. Reduction in sample thickness in the light path should increase delivery of UV dose at high sample hematocrits. In order to confirm this approach, several pressure drop measurements were undertaken using openings of varying dimensions. A pressure gauge was placed in line with a peristaltic pump both upstream and downstream from the narrowed openings. Whole blood of varying hematocrits was passed through the openings at controlled flow rates. Differences in the pressure readings at both locations permitted direct measurement of the pressure drop across the opening. Using this value and the dimensions of the opening, it was possible to determine the shear stress experienced by the red cells as they passed through the narrowed cell using the following equation:

$$\Delta P = \frac{8\mu l Q}{g d^3 w} \quad \text{Pressure Drop}$$

$$\tau_w = \frac{4\mu Q}{g w d^2} \quad \text{Shear Stress}$$

For blood,
μ = Viscosity = 0.0125/(1-Hematocrit)
g = gravitational constant = 981
Q = Flow Rate = mls/sec
1, w, d = Dimensions of opening in cm

Table 5: Measurement of Shear Stress on Red Cells As Functions of Flow Rate and Sample Hematocrit

| | | 0.08 X 0.008 | Dpmeas ($dynes/cm^2$) | 0.08 X 0010 | Dpmeas ($dynes/cm^2$) | 0.08 X 0.012 | Dpmeas ($dynes/cm^2$) |
|---|---|---|---|---|---|---|---|
| 41% HCT | Q=3.38 | 1.5 | 95.9 | 1.0 | 77.6 | 0.0 | 0.0 |
| 64% HCT | Q=3.38 | 4.0 | 255.8 | 3.0 | 232.9 | 2.0 | 182.1 |
| 41% HCT | Q=16.9 | 9.7 | 618.4 | 7.0 | 543.4 | 4.7 | 425.3 |
| 61% HCT | Q=16.9 | 20.7 | 1321.9 | 12.3 | 957.2 | 8.7 | 789.6 |

| | | 0.10 X 0.008 | Dpmeas ($dynes/cm^2$) | 0.1 X 0.010 | Dpmeas ($dynes/cm^2$) | 0.1 X 0.012 | Dpmeas ($dynes/cm^2$) |
|---|---|---|---|---|---|---|---|
| 41% HCT | Q=3.38 | 2.0 | 93.7 | 1.0 | 60.3 | 1.0 | 73.5 |
| 64% HCT | Q=3.38 | 4.5 | 210.8 | 3.0 | 180.9 | 2.0 | 146.9 |
| 41% HCT | Q=16.9 | 12.7 | 593.6 | 7.0 | 422.1 | 4.7 | 343.0 |
| 61% HCT | Q=16.9 | 23.3 | 1093.0 | 14.7 | 884.6 | 12.0 | 881.4 |

| | | 0.15 X 0.008 | Dpmeas ($dynes/cm^2$) | 0.15 X 0.010 | Dpmeas ($dynes/cm^2$) | 0.15 X 0.012 | Dpmeas ($dynes/cm^2$) |
|---|---|---|---|---|---|---|---|
| 41% HCT | Q=3.38 | 3.0 | 97.4 | 1.2 | 49.2 | 1.0 | 49.0 |
| 64% HCT | Q=3.38 | 6.5 | 211.0 | 3.5 | 143.5 | 2.0 | 97.9 |
| 41% HCT | Q=16.9 | 15.3 | 497.7 | 8.3 | 341.6 | 5.7 | 277.6 |
| 61% HCT | Q=16.9 | 35.7 | 1158.1 | 18.0 | 738.1 | 12.7 | 620.4 |

In previous experiments, it was determined that shear stresses of 1,000-2,000 dynes/$cm^2$ for intervals of 1-10 minutes or levels of 5,000-7,000 dynes/$cm^2$ for intervals of approximately 10 msec were sufficient to induce red cell hemolysis. Only in the case of the highest sample hematocrit (61%) and highest flow rate (16.9) did values exceed 1,000 dynes/$cm^2$. This occurred only for openings of the narrowest width (0.008 inches).

Values for the light penetration depth using the proposed configuration indicate that delivery in sufficient UV energy to drive virus inactivation processes is achievable even for samples with high hematocrits.

Results from shear stress analysis on red cell samples subjected to flow indicate that flow path dimensions may be significantly reduced and high flow rates maintained without risking red cell hemolysis.

**Preparation Example 8.**

A platelet concentrate was mixed with the platelet additive solution Isolyte S at a ratio of 20:80 platelet concentrate:Isolyte S. Mixtures of platelet concentrates and platelet additive solutions are referred to herein as in "media." Platelet concentrate without additive solution is referred to herein as in "plasma." Both were spiked with *Listeria monocytogenes.* Vitamin K5 was then added to each in the amount of 300 μg/mL B. Each was then exposed to UV, visible or room light in the cuvette apparatus of Figure 7 with the results shown in Table 6.

Table 6

| | Log Inactivation (cfu/mL) | |
|---|---|---|
| | **K5 in Media** | **K5 in Plasma** |
| **UV, 40 J/$cm^2$** | 4.2 Logs | 0.1 Logs |
| **VIS, 40 J/$cm^2$** | 4.2 Logs | 0.1 Logs |
| **Room Light** | 0 Logs | 0 Logs |
| UV Light = 365 nm<br>VIS Light=419 nm<br>Pathogen = *Listeria monocytogenes*<br>Concentration of K5 = 300 μg/mL | | |

**Preparation Example 9.**

Media and plasma as described above containing vitamin K5 were spiked with bacteria and irradiated or exposed to room light only (K5-light) as shown in Table 7, and growth evaluated after three days of incubation. Inactivation of some species was seen in the absence of irradiation.

Table 7

| | | **Media** | | **Plasma** | |
|---|---|---|---|---|---|
| | **Spike Level (cfu/mL)** | **K5 + Light** | **K5 - Light** | **K5 + Light** | **K5 - Light** |
| ***P. aeruginosa*** | *3.4 Logs* | - | - | - | - |
| ***S. aureus*** | *2.1 Logs* | - | - | + | + |
| ***S. epidermidis*** | *3.2 Logos* | - | + | - | - |
| ***L. monocytogenes*** | *3.5 Logs* | - | - | + | + |
| ***E. coli*** | *3.1 Logs* | - | - | + | - |
| UV Light = 365 nm, 40 J/$cm^2$<br>+ = Growth detected after three days incubation<br>- = No Growth detected after three days incubation<br>Concentration of K5 = 300 μg/mL | | | | | |

**Preparation Example 10.**

Media made using a platelet concentrate as described in Preparation Example 8 and Isolyte S at a ratio of

Isolyte S:platelet concentrate of 70:30 and containing 300 μg/mL vitamin K5 was spiked with several species of bacteria and irradiated at energy levels of 30 and 60 J/cm$^2$. Inactivation as a function of energy of irradiation is set forth in Table 8 and Figure 8.

Table 8

| Energy (J/cm$^2$) | *S. aureus* | *S. epidermidis* | *L. monocytogenes* | *E. coli* |
|---|---|---|---|---|
| 0 | 4.3 | 2.6 | 2.8 | 3.5 |
| 30 | 3.6 | 2.7 | 2 | 2 |
| 60 | 3.2 | 2.5 | 1 | 1 |

**Example 11.**

To platelet concentrate as described in Preparation Example 8 and to 70:30 media as described in Preparation Example 10 was added 10 μM of 7,8-dimethyl-10-ribityl isoalloxazine. The platelet concentrate and media were spiked with *S. aureus* or *S. epidermidis,* and irradiated at 80 J/cm$^2$ and 30 J/cm$^2$ and inactivation measured as above. Results are shown in Figure 9.

**Example 12.**

To plasma concentrate as described in Preparation Example 8 contained in a standard blood bag was added 25 μM 7,8-dimethyl-10-ribityl isoalloxazine in powder form. The bag was spiked with bacteria as shown in Table 9, agitated and exposed to 120 J/cm$^2$ radiation. Inactivation results are set forth in Table 9.

Table 9

| Pathogen | Log Inactivation (cfu/mL) |
|---|---|
| *S. aureus* | 1.7 Logs |
| *S. epidermidis* | 3.5 Logs |
| *P. aeruginosa* | 3.6 Logs |
| *E. coli* | 4.1 Logs |

**Example 13.**

To platelet concentrate as described in Preparation Example 8 was added 7,8-dimethyl-10-ribityl isoalloxazine, alloxazine mononucleotide, or 7-8-dimethyl alloxazine, followed by spiking with *S. aureus* or *S. epidermidis,* and irradiation at 80 J/cm$^2$. Inactivation results are shown in Table 10.

Table 10

| | Log Inactivation (cfu/mL) | |
|---|---|---|
| | *Staphylococcus aureus* | *Staphylococcus epidermidis* |
| **7,8-dimethyl-10-ribityl isoalloxazine, 10 μM** | 1.9 Logs | 4.1 Logs |
| **alloxazine mononucleotide, 10 μM** | 1.6 Logs | 5.6 Logs |
| **7-8-dimethyl alloxazine, 7μM** | 1.6 Logs | 2.9 Logs |

**Example 14.**

To platelet concentrate of Preparation Example 8 was added 10 μM 7,8-dimethyl-10-ribityl-isoalloxazine. Aliquots contained no additive, 10 mM ascorbate or 10 mM KI as a "quencher" or antioxidant. The solutions were spiked with HSV-2, ΦX174, *S. epidermidis* or *S. aureus* and irradiated at 80 J/cm$^2$. Results are shown in Figure 10.

**Example 15.**

To platelet concentrates of Preparation Example 8 were added varying concentrations of 7,8-dimethyl-10-ribityl-isoalloxazine. These solutions were spiked with herpes simplex virus type II (HSV-II), a double-stranded DNA envelope virus. Irradiation was done at 80 J/cm$^2$. The experiment was replicated three times. In all three trials complete inactivation was achieved. Results are shown in Figure 11.

**Example 16.**

The protocol of Example 15 was followed using *S. epidermidis* instead of HSV II at energies of irradiation of 40, 80 and 120 J/cm$^2$. Inactivation results are shown in Figure 12.

**Example 17.**

The protocol of Example 15 was followed using ΦX174, a single stranded DNA bacteriophage, at varying concentrations of 7,8-dimethyl-10-ribityl-isoalloxazine and energies of irradiation. Inactivation results are shown in Figure 13.

**Example 18.**

To platelet concentrates of Preparation Example 8 was added 10 μM 7,8-dimethyl-10-ribityl-isoalloxazine. These were spiked with *S. aureus* or ΦX174 and irradiated at varying energies of irradiation with a 50:50 mixture of visible and ultraviolet light. Inactivation results are shown in Figure 14.

**Example 19.**

The protocol of Example 18 was followed using *S. epidermidis* and HSV-II as the microorganisms. A 50:50 mixture of ultraviolet and visible light was supplied by DYMAX light source. Inactivation results are shown in Figure 15.

**Example 20.**

To platelet concentrate of Preparation Example 8 was added 10 μM 7,8-dimethyl-10-ribityl-isoalloxazine in powdered form. Tests with and without added ascorbate were conducted. 150 ml of the test solutions were placed in a Spectra™ blood bag and shaken and exposed to varying energies of irradiation using 50:50 visible:ultraviolet light. After receiving 40 J/cm$^2$, the contents of each bag were transferred to a new bag to avoid errors due to microorganisms which may have remained in the spike port of the bag. Inactivation results are shown in Figure 16. Downward arrows indicate inactivation to the level it was possible to detect (2.5 log titre).

**Example 21.**

To platelet concentrate of Preparation Example 8 and platelet concentrate in Isolyte S at 30:70 platelet concentrate:Isolyte S, was added 20 μM 7,8-dimethyl-10-ribityl-isoalloxazine. These were spiked with vaccinia virus, a double stranded DNA envelope virus, and exposed to 60 J/cm$^2$ visible light or mixed (50:50) visible and ultraviolet light using a DYMAX 2000 UV light source for 30 minutes. The limit of detection was 1.5 logs. Inactivation results are show in Figure 17. Comparisons were done using no photosensitizer, photosensitizer in Isolyte S media alone, platelets in Isolyte S media, platelets in Isolyte S media using 8-methoxy psoralen instead of 7,8-dimethyl-10-ribityl-isoalloxazine, and platelet concentrate in Isolyte media (30:70).

**Example 22.**

Samples of platelet concentrate in Isolyte S media 30:70, with and without 10 μM 7,8-dimethyl-10-ribityl-isoalloxazine were spiked with vaccinia virus and irradiated at 60 J/cm$^2$ with 50:50 visible:UV light for varying periods of time and inactivation results compared as shown in Figure 18.

**Example 23.**

To samples of platelet concentrate as described in Preparation Example 8 were added 5 μM or 50 μM 7,8-dimethyl-10-ribityl-isoalloxazine. Samples were spiked with HIV 1. Using the cuvette flow cell shown in Figure 7, samples

were irradiated with 50:50 visible:UV light at varying energies using an EFOS light system. Inactivation results are show in Figure 19.

**Example 24.**

HIV-infected ACH-2 cells were added to samples of platelet concentrate described in Preparation Example 8. 5 or 50 μM of 7,8-dimethyl-10-ribityl-isoalloxazine were added to the samples. The protocol of Example 23 was followed, and inactivation results are shown in Figure 20. The presence of HIV was assayed by its cytopathic effect on test cells.

**Example 25.**

The protocol of Example 24 was followed and the presence of HIV assayed by quantifying the level of P24 antigen production. Inactivation results are show in Figure 21.

**Example 26.**

To samples of platelet concentrate as described in Preparation Example 8 and media containing 30% platelet concentrate and 70% PASIII™ media were added 6 mM ascorbate and 14 μM 7,8-dimethyl-10-ribityl-isoalloxazine. Samples were spiked with HSV-II. Inactivation results are show in Figure 22 and Table 11.

Table 11

| Time (Minutes) | Energy (UV+VIS) J/cm$^2$ | 30:70 PC:Media log virus titre | Energy (UV+VIS) J/cm$^2$ | 90:10 PC:Media log virus titre |
|---|---|---|---|---|
| 0 | 0 | 5.6 | 0 | 5.6 |
| 1.5 | 5 | 2.5 | 40 | 3.3 |
| 3 | 10 | 2.5 | 80 | 1.5 No Detectable Virus |
| 4.5 | 15 | 2.3 | 120 | 1.5 No Detectable Virus |
| 6 | 20 | 1.8 | | |
| 9 | 30 | 1.6 | | |
| 12 | 40 | | | |
| 24 | 80 | | | |
| 36 | 120 | | | |

**Claims**

**1.** A method for treating a fluid *in vitro* to inactive microorganisms which may be present therein, wherein said fluid contains one or more components selected from whole blood, biologically active proteins derived from blood or blood constituents, red blood cells, platelets, plasma and therapeutic protein compositions containing proteins derived from blood, said method comprising:

(a) adding an inactivation-effective amount of a photosensitizer which is an endogenous alloxazine or endogenously-based alloxazine derivative to said fluid;
(b) exposing the fluid of step (a) to ultraviolet light, whereby said microorganisms are inactivated.

**2.** A method according to claim 1 wherein the photosensitizer is added to said fluid in a non-toxic amount.

**3.** A method of claim 1 or 2, wherein said fluid comprises blood components separated from whole blood.

**4.** The method of one of claims 1 to 3 wherein said photosensitizer is an endogenous alloxazine photosensitizer selected from 7,8-dimethyl-10-ribityl isoalloxazine, 7,8-dimethylalloxazine, 7,8,10-trimethylisoalloxazine, alloxazine

mononucleotide and isoalloxazine-adenosine dinucleotide.

5. The method of one of claims 1 to 3 wherein said microorganisms are selected from HIV viruses, hepatitis viruses, sindbis virus, cytomegalovirus, vesicular stomatitis virus, herpes simplex viruses, vaccinia virus, human T-lymphotropic retroviruses, HTLV-III, lymphadenopahy virus LAV/IDAV, parvovirus, transfusion-transmitted (TT) virus, Epstein-Barr virus, bacteriophages ΦX174, Φ6, λ, R17, $T_4$, $T_2$, *P. aeruginosa, S. aureus, S. epidermidis, L. monocytogenes, E. coli, K. pneumoniae and S. marcescens.*

6. The method of one of claims 1 to 3 which further comprises flowing the fluid containing said photosensitizer past a source of photoradiation at a rate and depth selected to ensure penetration of the photoradiation through the fluid and inactivation of the microorganisms, or containing said fluid and photosensitizer in a container transparent to said photoradiation and exposing said fluid to said photoradiation.

7. The method of one of claims 1 to 3 wherein said photosensitizer is added to anticoagulant and said anticoagulant is added to said fluid.

8. A method of one of claims 1 to 3 wherein an enhancer is added to said fluid prior to exposing said fluid to photoradiation.

**Patentansprüche**

1. Verfahren zum Behandeln eines Fluids *in vitro* zur Inaktivierung von Mikroorganismen, welche darin vorhanden sein können, wobei das Fluid eine oder mehrere Komponenten enthält, die aus Vollblut, von Blut oder Blutbestandteilen abgeleiteten biologisch aktiven Proteinen, roten Blutkörperchen, Plättchen, Plasma und therapeutischen Proteinzusammensetzungen, welche von Blut abgeleitete Proteine enthalten, ausgewählt sind, wobei das Verfahren umfasst:

   (a) das Geben einer für Inaktivierung wirksamen Menge eines Photosensibilisators, welcher ein endogenes Alloxazin oder ein Alloxazin-Derivat auf der Basis eines endogenen Alloxazins ist, zu dem Fluid;
   (b) das Bestrahlen des Fluids von Schritt (a) mit ultraviolettem Licht, wobei die Mikroorganismen inaktiviert werden.

2. Verfahren gemäß Anspruch 1, wobei der Photosensibilisator zu dem Fluid in einer nicht toxischen Menge gegeben wird.

3. Verfahren nach Anspruch 1 oder 2, wobei das Fluid von Vollblut abgetrennte Blutkomponenten umfasst.

4. Verfahren nach einem der Ansprüche 1 bis 3, wobei der Photosensibilisator ein endogener Alloxazin-Photosensibilisator ist, welcher aus 7,8-Dimethyl-10-ribitylisoalloxazin, 7,8-Dimethylalloxazin, 7,8,10-Trimethylisoalloxazin, Alloxazinmononucleotid und Isoalloxazinadenosindinucleotid ausgewählt ist.

5. Verfahren nach einem der Ansprüche 1 bis 3, wobei die Mikroorganismen aus HIV-Viren, Hepatitis-Viren, Sindbisvirus, Zytomegalovirus, aphthöse-Stomatitis-Virus, Herpes simplex-Viren, Vaccinia-Virus, humanen T-lymphotropen Retroviren, HTLV-III, Lymphadenopathie-Virus LAV/IDAV, Parvovirus, durch Transfusion übertragenem (TT) Virus, Epstein-Barr-Virus, Bakteriophagen ΦX174, Φ6, λ, R17, $T_4$, $T_2$, *P. aeruginosa, S. aureus, S. epidermidis, L. monocytogenes, E. coli, K. pneumoniae* und *S. marcescens* ausgewählt sind.

6. Verfahren nach einem der Ansprüche 1 bis 3, welches ferner das Vorbeifließen des Fluids, das den Photosensibilisator enthält, an einer Photostrahlungsquelle in einer Geschwindigkeit und Tiefe, welche so gewählt sind, dass eine Penetration der Photostrahlung durch das Fluid und eine Inaktivierung der Mikroorganismen sichergestellt werden, oder das Enthalten des Fluids und Photosensibilisators in einem Behälter, welcher für die Photostrahlung transparent ist, und das Bestrahlen des Fluids mit der Photostrahlung umfasst.

7. Verfahren nach einem der Ansprüche 1 bis 3, wobei der Photosensibilisator zu einem Antikoagulans gegeben wird und das Antikoagulans zu dem Fluid gegeben wird.

8. Verfahren nach einem der Ansprüche 1 bis 3, wobei ein Enhancer zu dem Fluid gegeben wird, bevor das Fluid mit Photostrahlung bestrahlt wird.

## Revendications

1. Procédé pour traiter un fluide *in vitro* afin d'inactiver des microorganismes qui peuvent y être présents, dans lequel ledit fluide contient un ou plusieurs composants choisis parmi le sang total, des protéines biologiquement actives issues de sang ou de constituants sanguins, des globules rouges, des plaquettes, du plasma et des compositions thérapeutiques à base de protéines contenant des protéines issues de sang, ledit procédé comprenant :

   (a) l'addition audit fluide d'une quantité, efficace pour l'inactivation, d'un agent photosensibilisant qui est une alloxazine endogène ou un dérivé d'alloxazine d'origine endogène ;
   (b) l'exposition du fluide de l'étape (a) à la lumière ultraviolette, ce qui permet d'inactiver lesdits microorganismes.

2. Procédé selon la revendication 1, dans lequel l'agent photosensibilisant est ajouté audit fluide en une quantité non toxique.

3. Procédé selon la revendication 1 ou 2, dans lequel ledit fluide comprend des composants sanguins séparés du sang total.

4. Procédé selon l'une des revendications 1 à 3, dans lequel ledit agent photosensibilisant est un agent photosensibilisant endogène à base d'alloxazine choisi parmi la 7,8-diméthyl-10-ribityl-isoalloxazine, la 7,8-diméthyl-alloxazine, la 7,8,10-triméthylisoalloxazine, un mononucléotide alloxazine et un dinucléotide isoalloxazine-adénosine.

5. Procédé selon l'une des revendications 1 à 3, dans lequel lesdits microorganismes sont choisis parmi les virus VIH, les virus de l'hépatite, le virus Sindbis, le cytomégalovirus, le virus de la stomatite vésiculeuse, les virus herpes simplex, le virus de la vaccine, les rétrovirus T-lymphotropiques humains, HTLV-III, le virus de la lymphadénopathie associé à la lymphadénopathie/à l'immunodéficience (LAV/IDAV), le parovirus, le virus transmis par transfusion (TT), le virus Epstein-Barr, les bactériophages ΦX174, Φ6, λ, R17, $T_4$, $T_2$, *P. aeruginosa, S. aureus, S. epidermidis, L. monocytogenes, E. coli, K. pneumoniae* et *S. marcescens.*

6. Procédé selon l'une des revendications 1 à 3, comprenant en outre l'écoulement du fluide contenant ledit agent photosensibilisant au travers d'une source de rayonnement de lumière à un débit et une profondeur choisis pour assurer la pénétration du rayonnement de lumière au travers du fluide et l'inactivation des microorganismes, ou contenant lesdits fluide et agent photosensibilisant dans un récipient transparent audit rayonnement de lumière et permettant d'exposer ledit fluide audit rayonnement de lumière.

7. Procédé selon l'une des revendications 1 à 3, dans lequel ledit agent photosensibilisant est ajouté à un anticoagulant, et ledit anticoagulant est ajouté audit fluide.

8. Procédé selon l'une des revendications 1 à 3, dans lequel un renforçateur est ajouté audit fluide avant exposition dudit fluide au rayonnement de lumière.

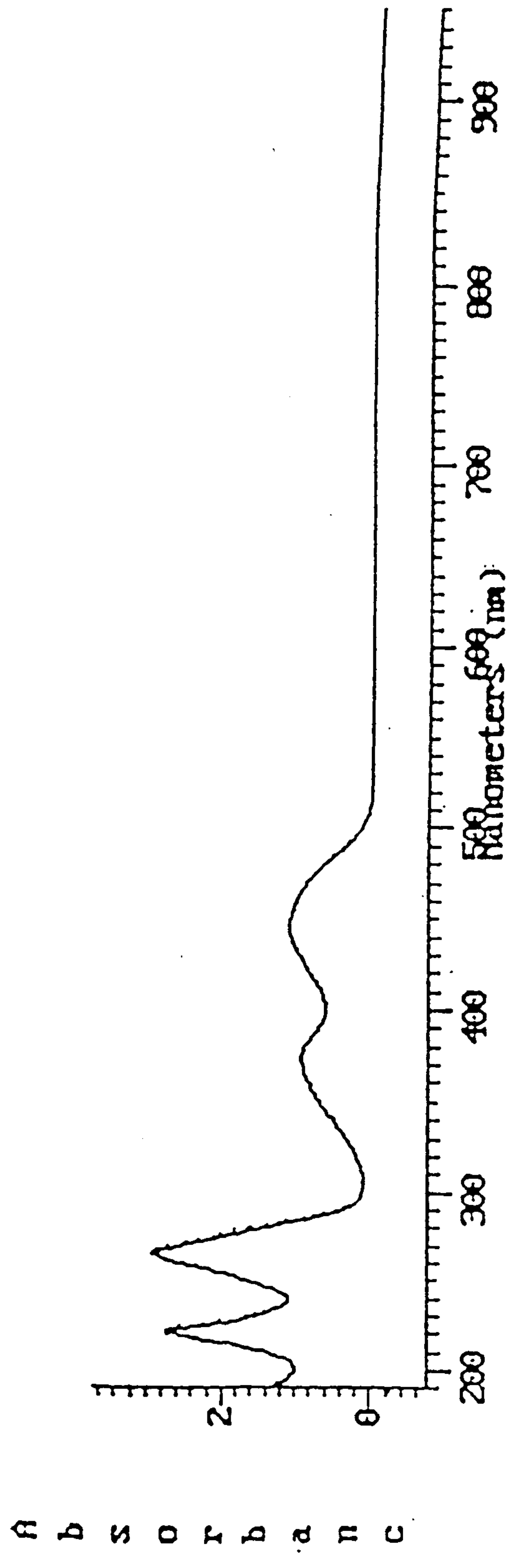


FIG. 1

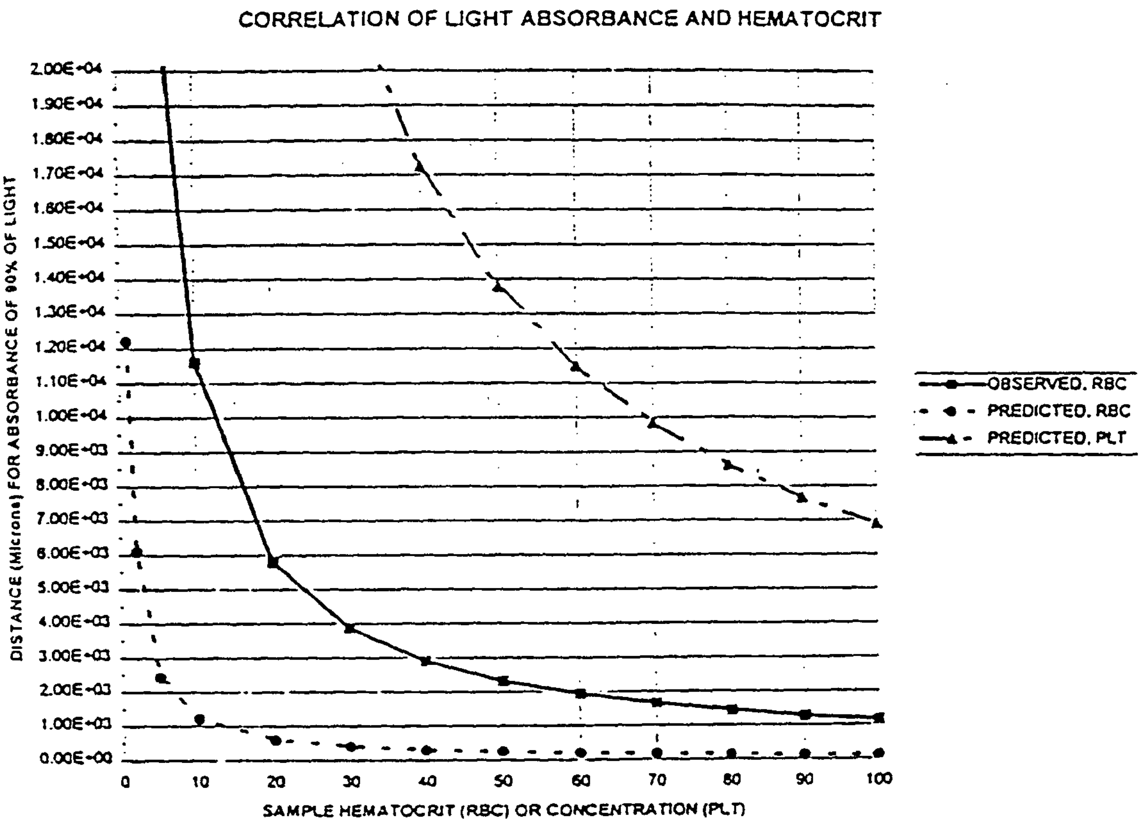
CORRELATION OF LIGHT ABSORBANCE AND HEMATOCRIT
2.00E+04
1.90E+04
1.80E+04
1.70E+04
1.60E+04
1.50E+04
1.40E+04
1.30E+04
1.20E+04
1.10E+04
1.00E+04
9.00E+03
8.00E+03
7.00E+03
6.00E+03
5.00E+03
4.00E+03
3.00E+03
2.00E+03
1.00E+03
0.00E+00
DISTANCE (Microns) FOR ABSORBANCE OF 90% OF LIGHT
0
10
20
30
40
50
60
70
80
90
100
SAMPLE HEMATOCRIT (RBC) OR CONCENTRATION (PLT)
OBSERVED. RBC
PREDICTED. RBC
PREDICTED. PLT

FIG. 2

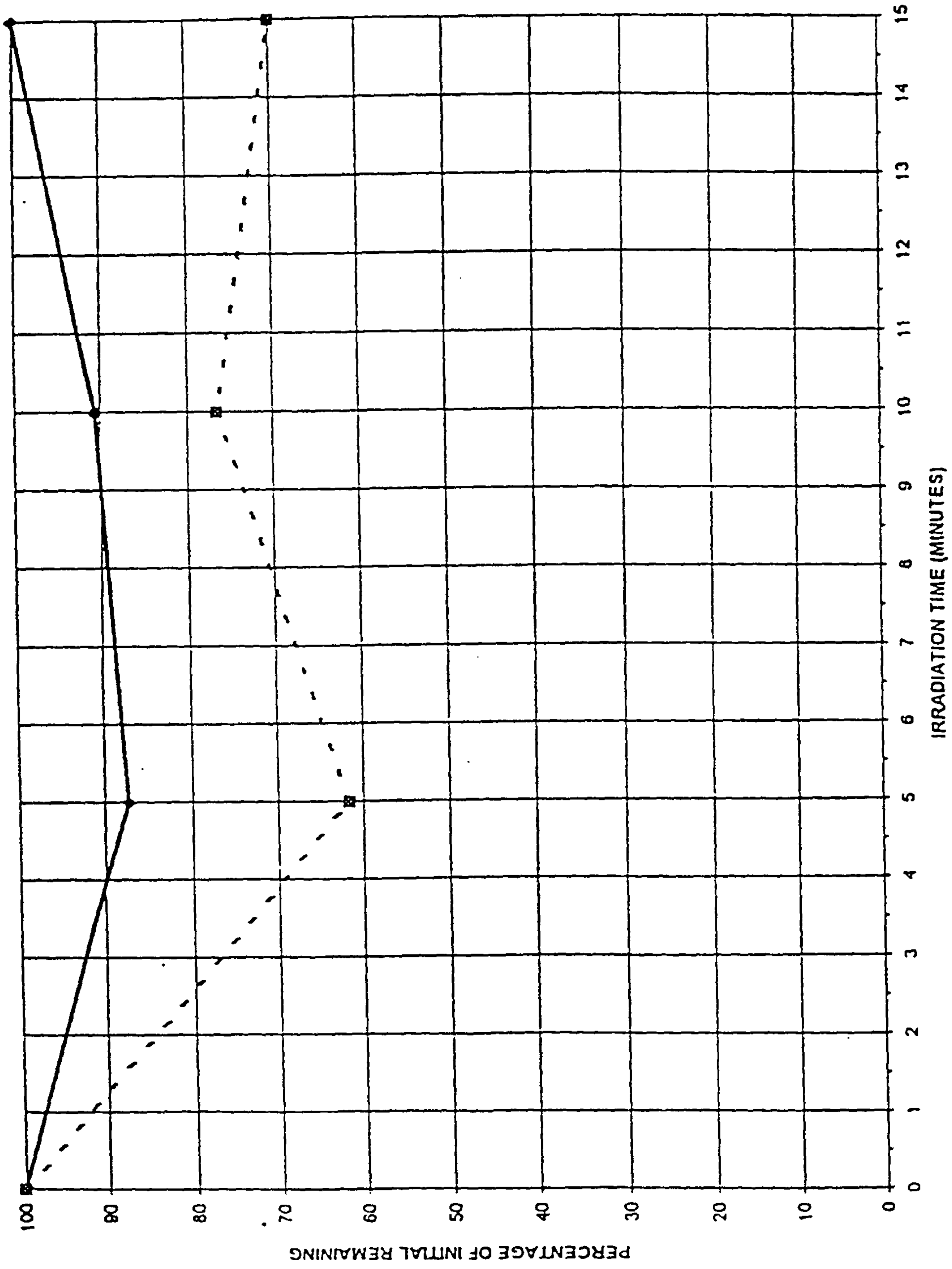

IRRADIATION TIME (MINUTES)
0 1 2 3 4 5 6 7 8 9 10 11 12 13 14 15
PERCENTAGE OF INITIAL REMAINING
100 90 80 70 60 50 40 30 20 10 0


FIG. 3

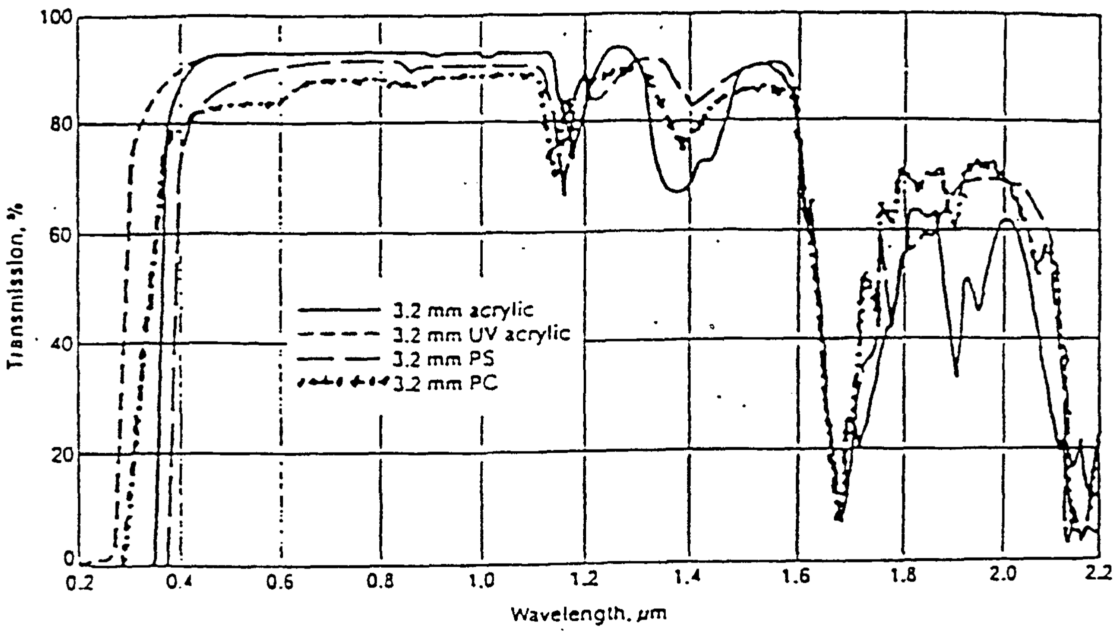
100
80
60
40
20
0
Transmission, %
0.2
0.4
0.6
0.8
1.0
1.2
1.4
1.6
1.8
2.0
2.2
Wavelength, μm
3.2 mm acrylic
3.2 mm UV acrylic
3.2 mm PS
3.2 mm PC

FIG. 4

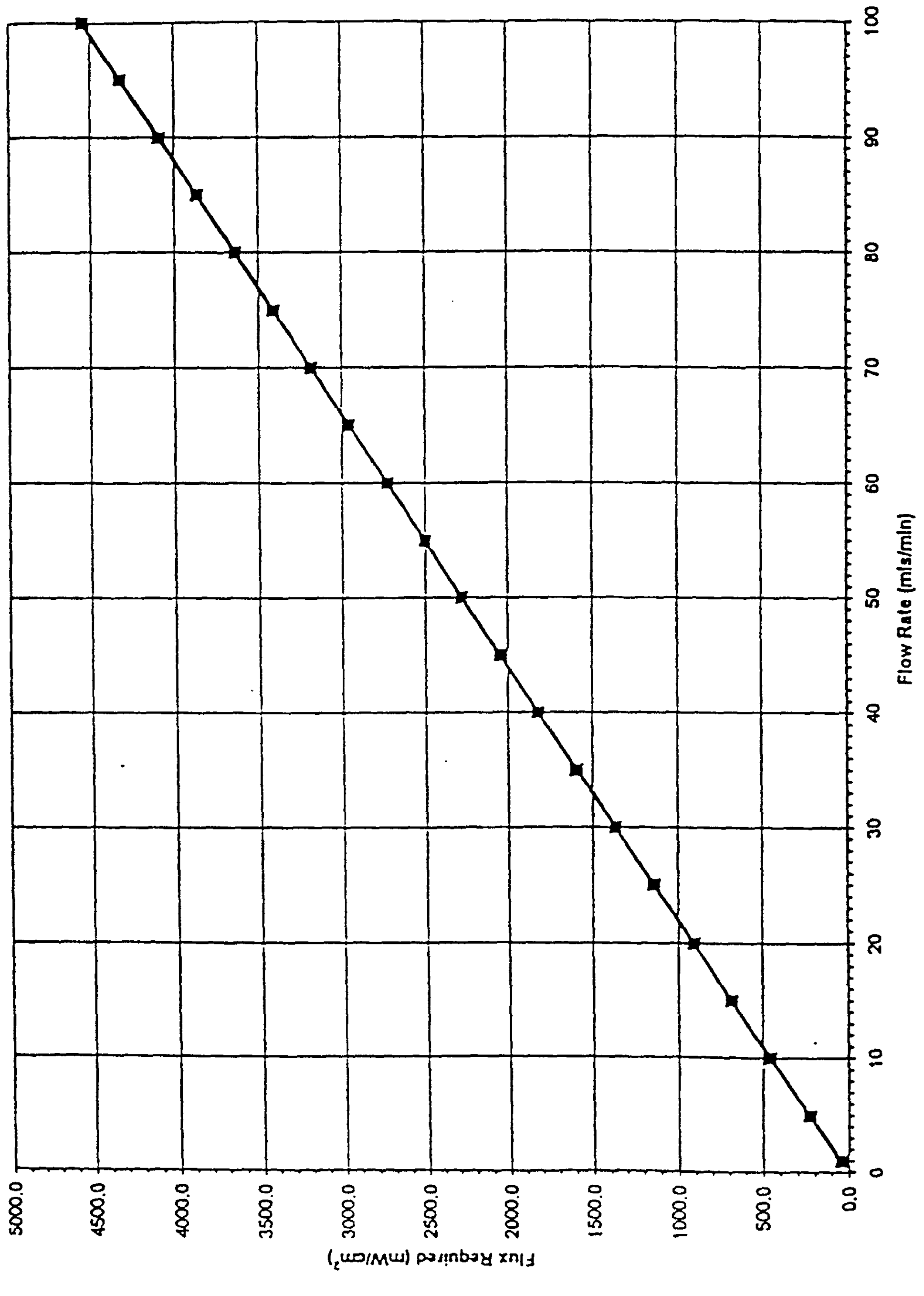
Flux Required (mW/cm²)
5000.0
4500.0
4000.0
3500.0
3000.0
2500.0
2000.0
1500.0
1000.0
500.0
0.0
Flow Rate (mls/min)
0
10
20
30
40
50
60
70
80
90
100

FIG. 5

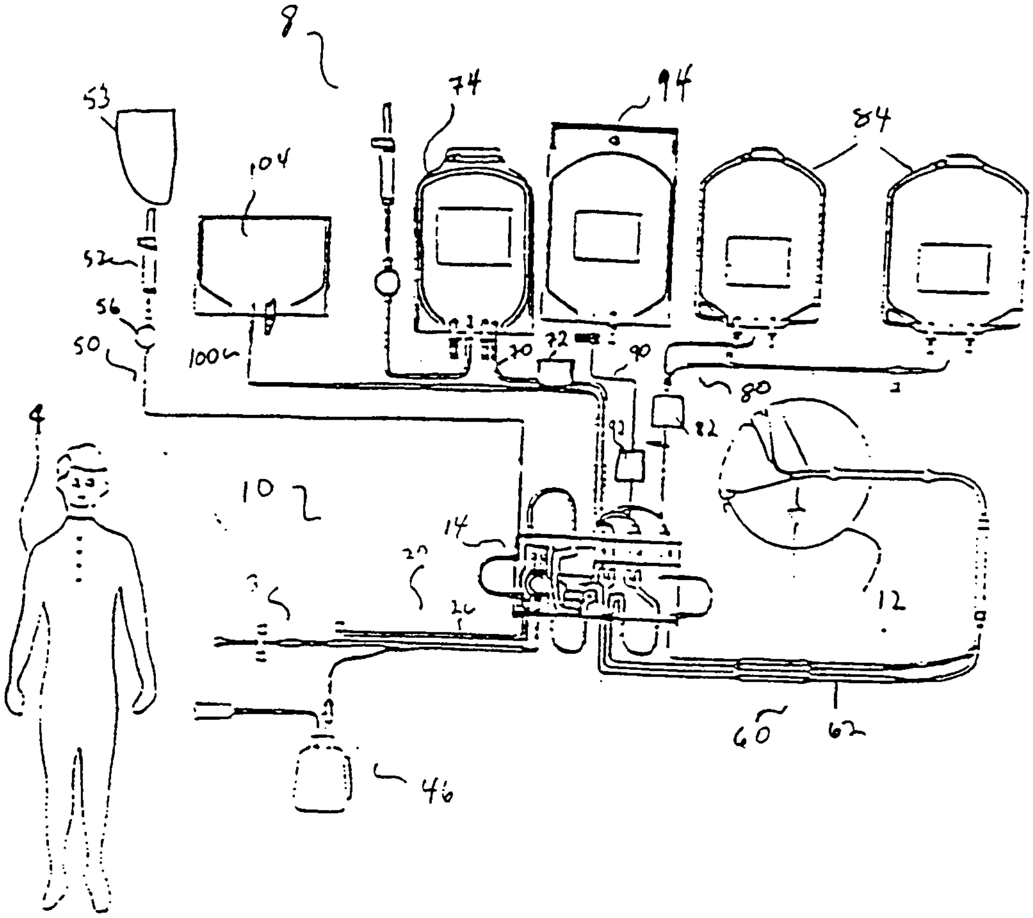
8
53
104
74
94
84
52
56
50
100
70
72
90
80
82
92
4
10
14
12
20
26
60
62
46

FIG. 6

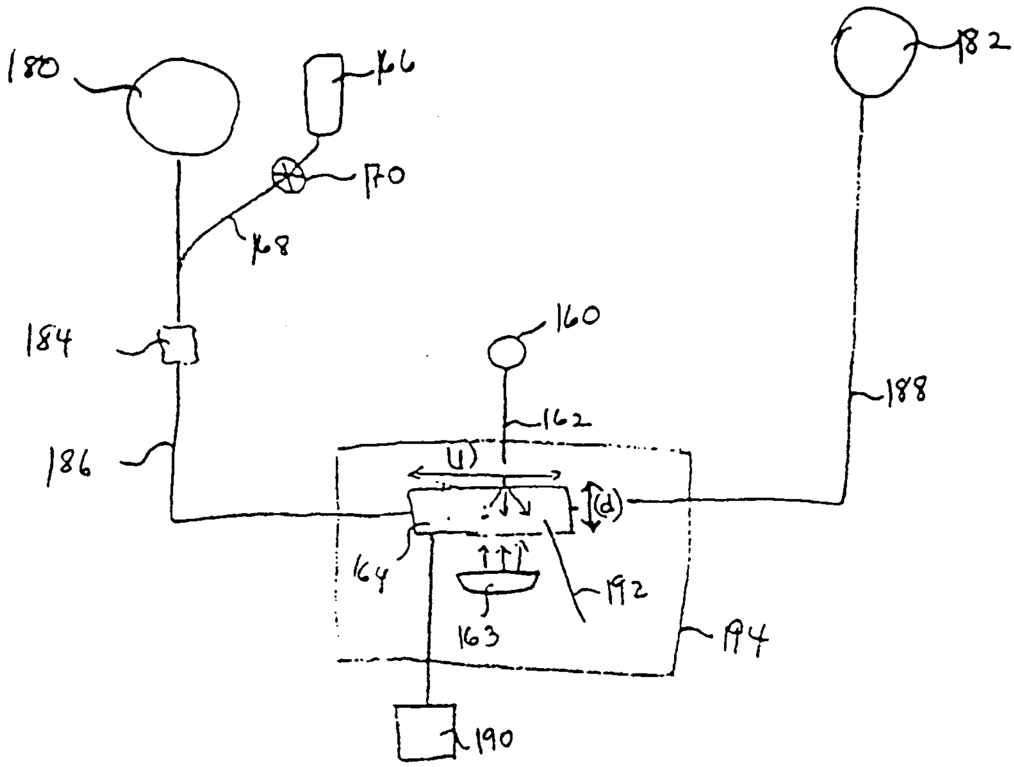


FIG. 7

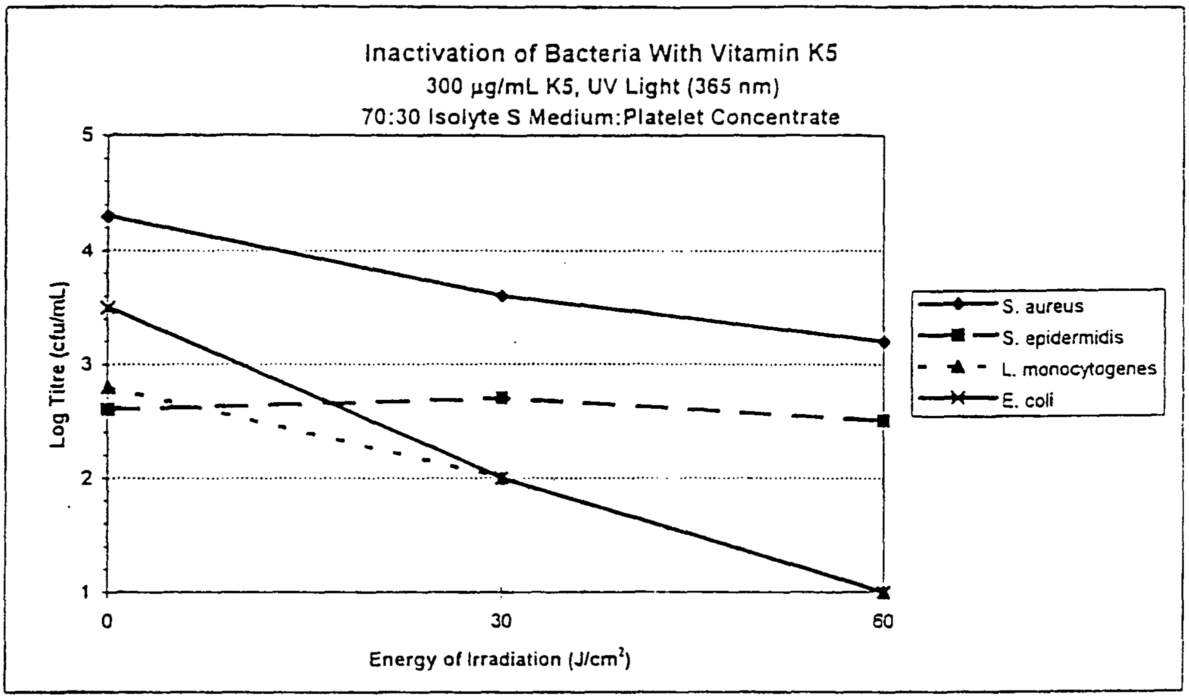
Inactivation of Bacteria With Vitamin K5
300 µg/mL K5, UV Light (365 nm)
70:30 Isolyte S Medium:Platelet Concentrate
Log Titre (cfu/mL)
5
4
3
2
1
0
30
60
Energy of Irradiation (J/cm²)
S. aureus
S. epidermidis
L. monocytogenes
E. coli

FIG. 8

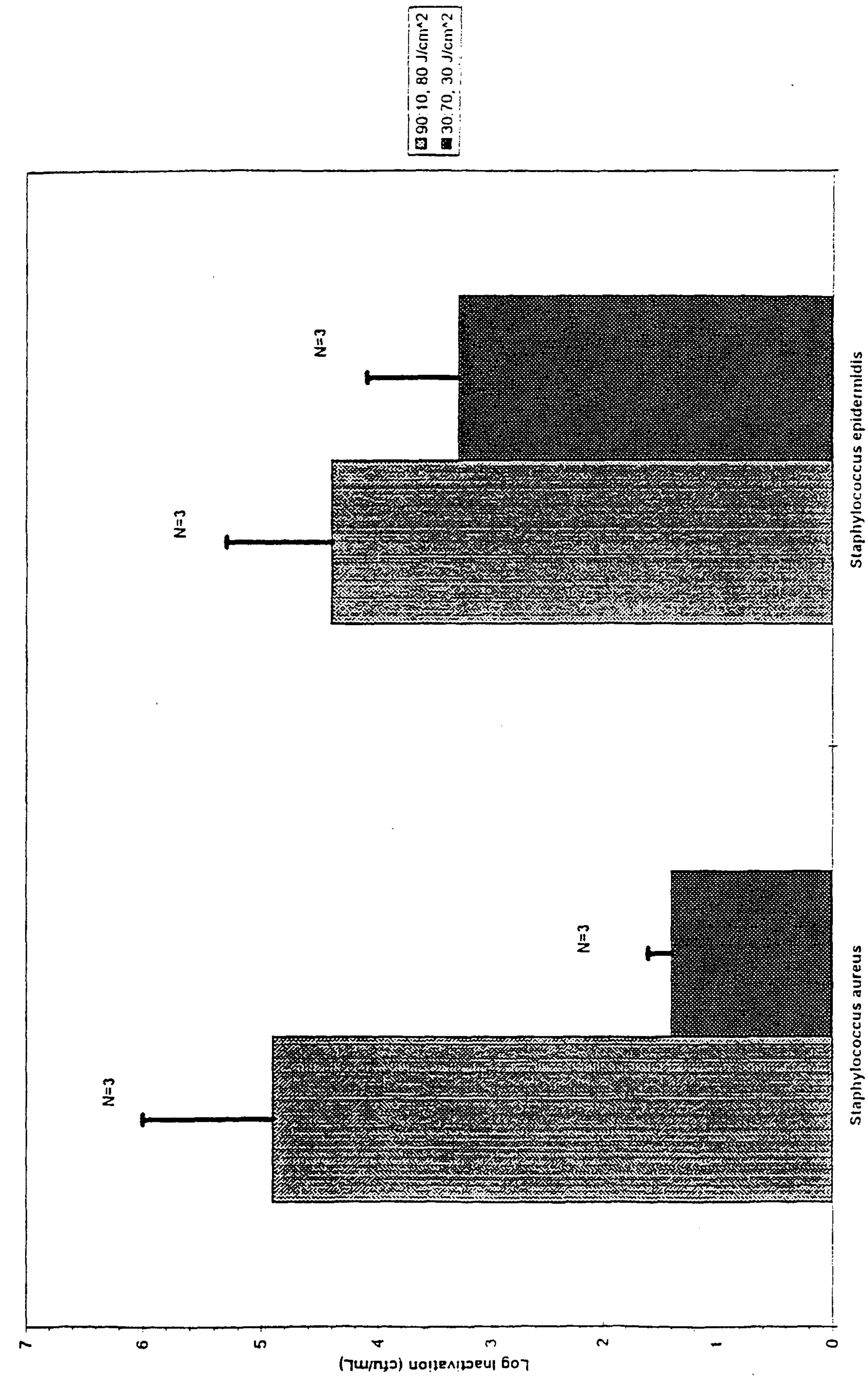
90:10, 80 J/cm^2
30:70, 30 J/cm^2
N=3
N=3
N=3
N=3
Staphylococcus aureus
Staphylococcus epidermidis
Log inactivation (cfu/mL)
0
1
2
3
4
5
6
7

FIG. 9

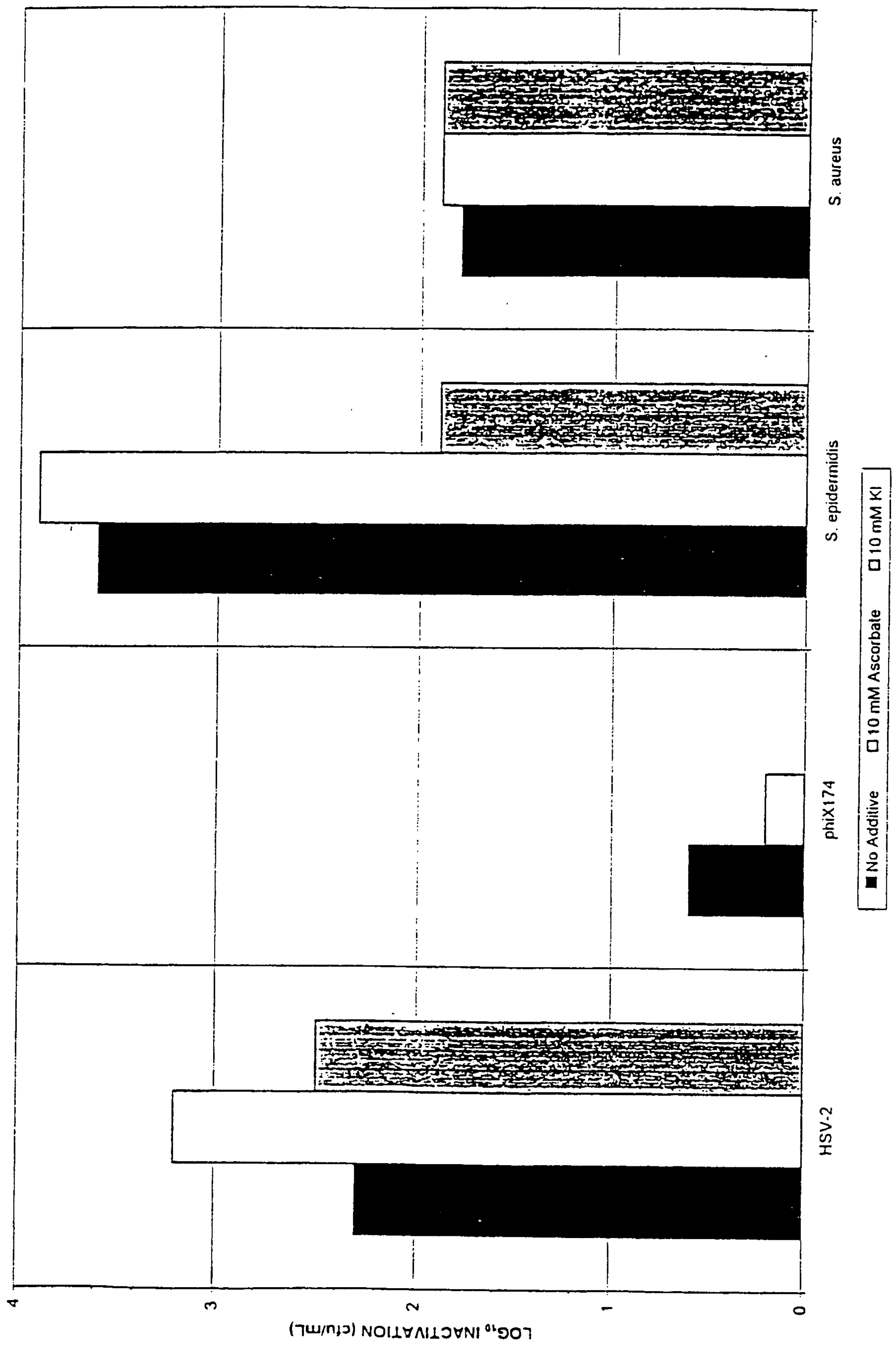
LOG$_{10}$ INACTIVATION (cfu/mL)
0
1
2
3
4
HSV-2
phiX174
S. epidermidis
S. aureus
No Additive
10 mM Ascorbate
10 mM KI

FIG. 10

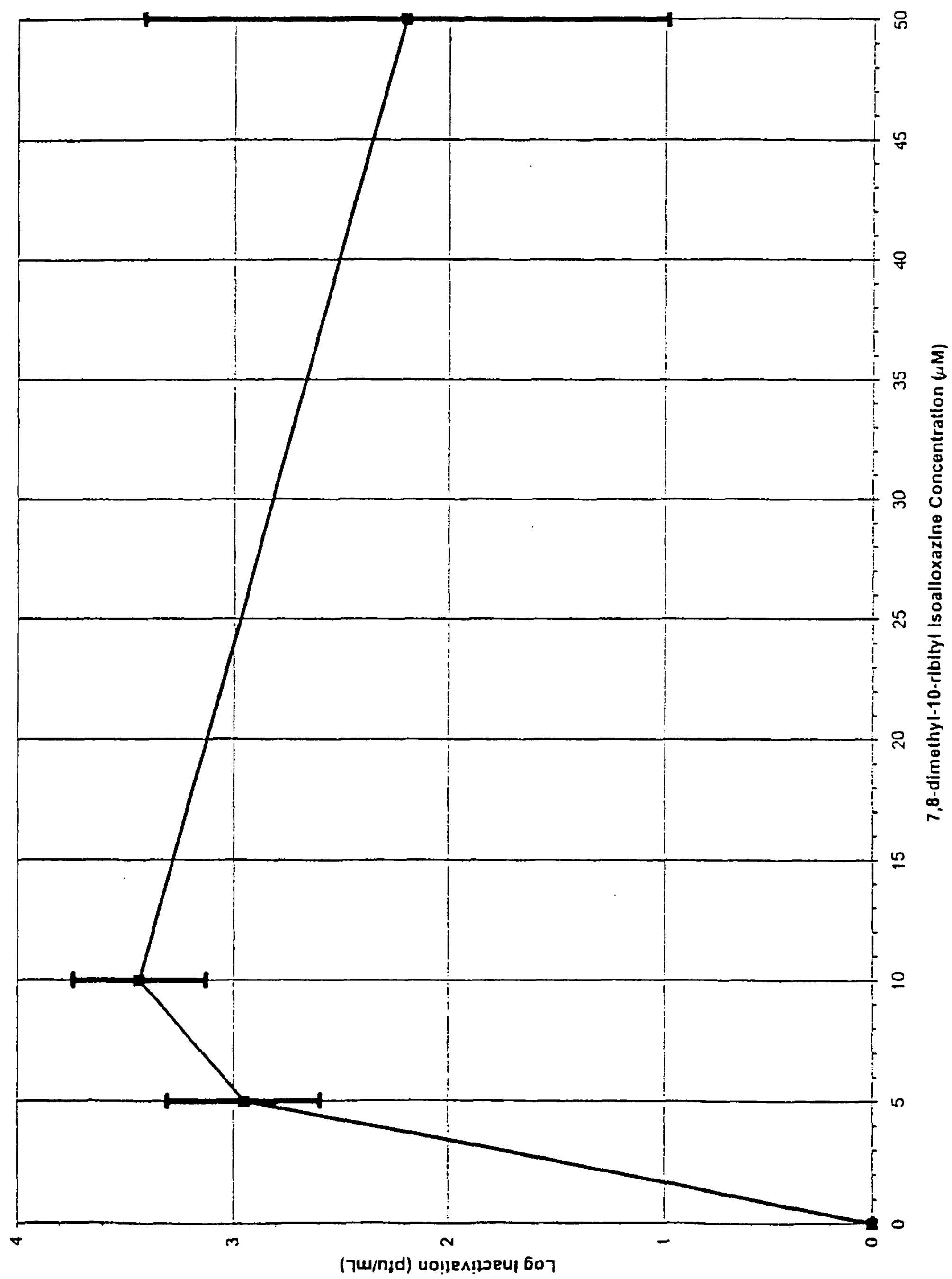
Log Inactivation (pfu/mL)
0
1
2
3
4
0
5
10
15
20
25
30
35
40
45
50
7,8-dimethyl-10-ribityl Isoalloxazine Concentration (μM)

FIG. 11

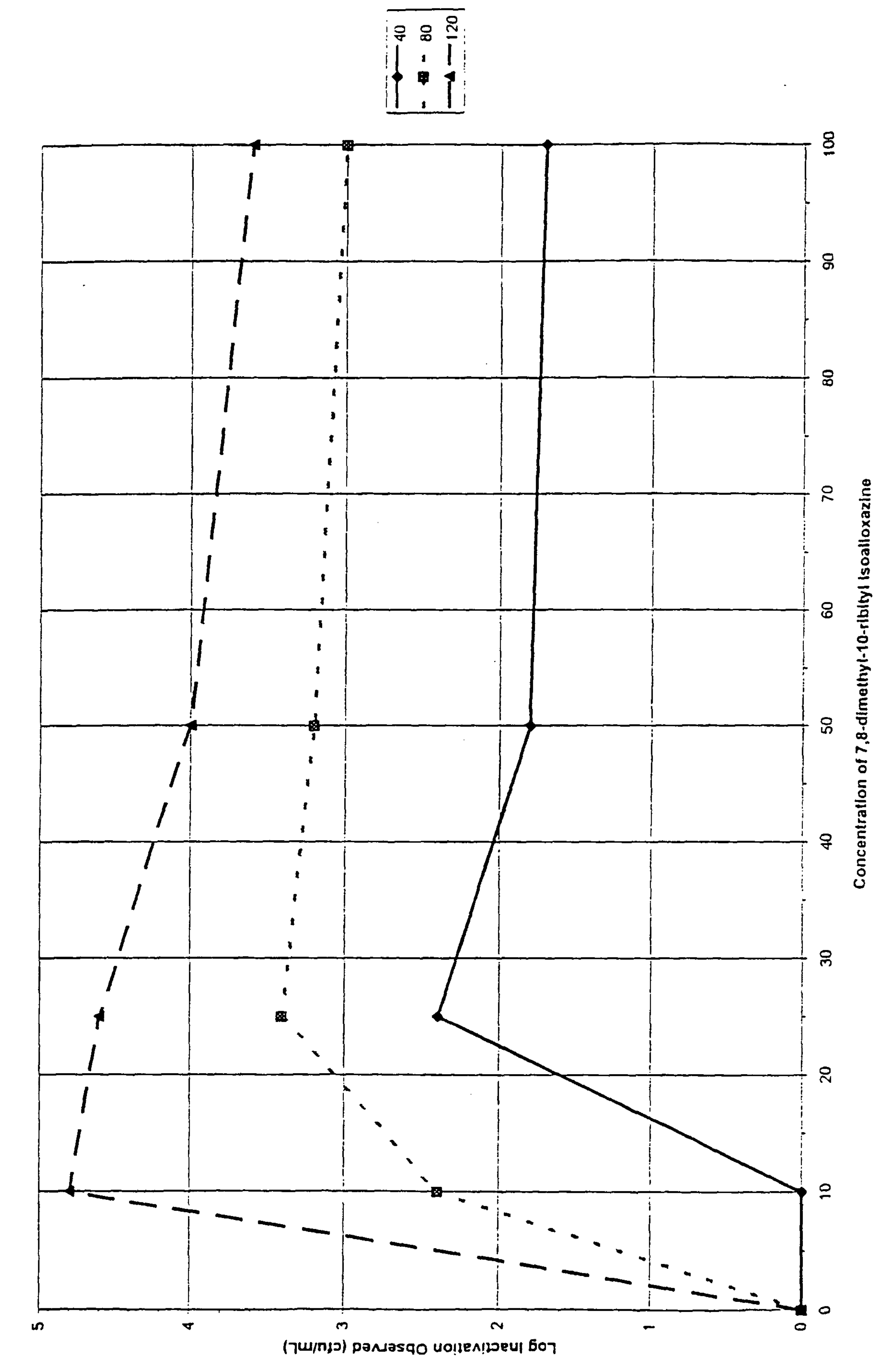
40
80
120
Log Inactivation Observed (cfu/mL)
0
1
2
3
4
5
Concentration of 7,8-dimethyl-10-ribityl isoalloxazine
0
10
20
30
40
50
60
70
80
90
100

FIG. 12

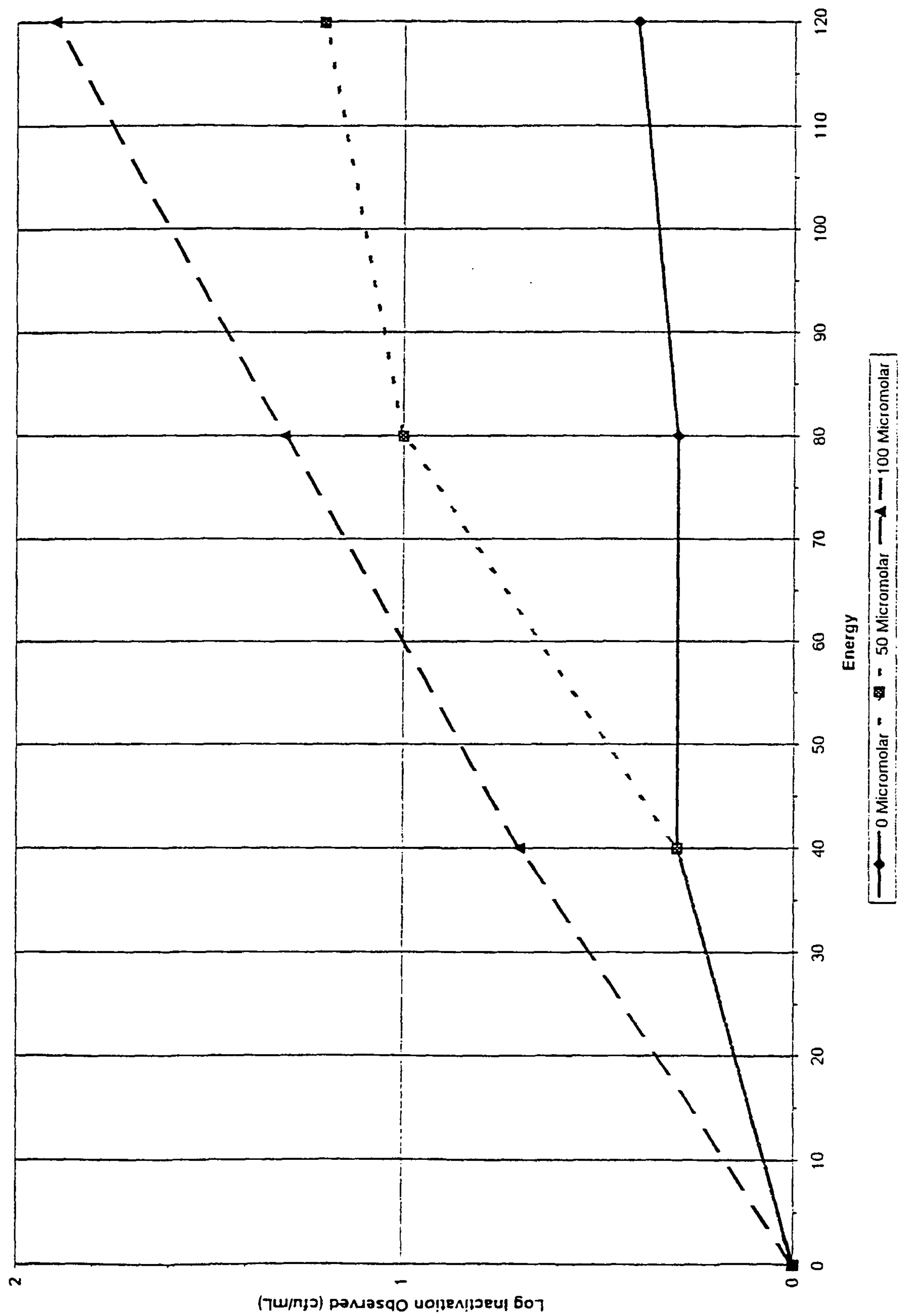

Log Inactivation Observed (cfu/mL)
0
1
2
0
10
20
30
40
50
60
70
80
90
100
110
120
Energy
0 Micromolar
50 Micromolar
100 Micromolar


FIG. 13

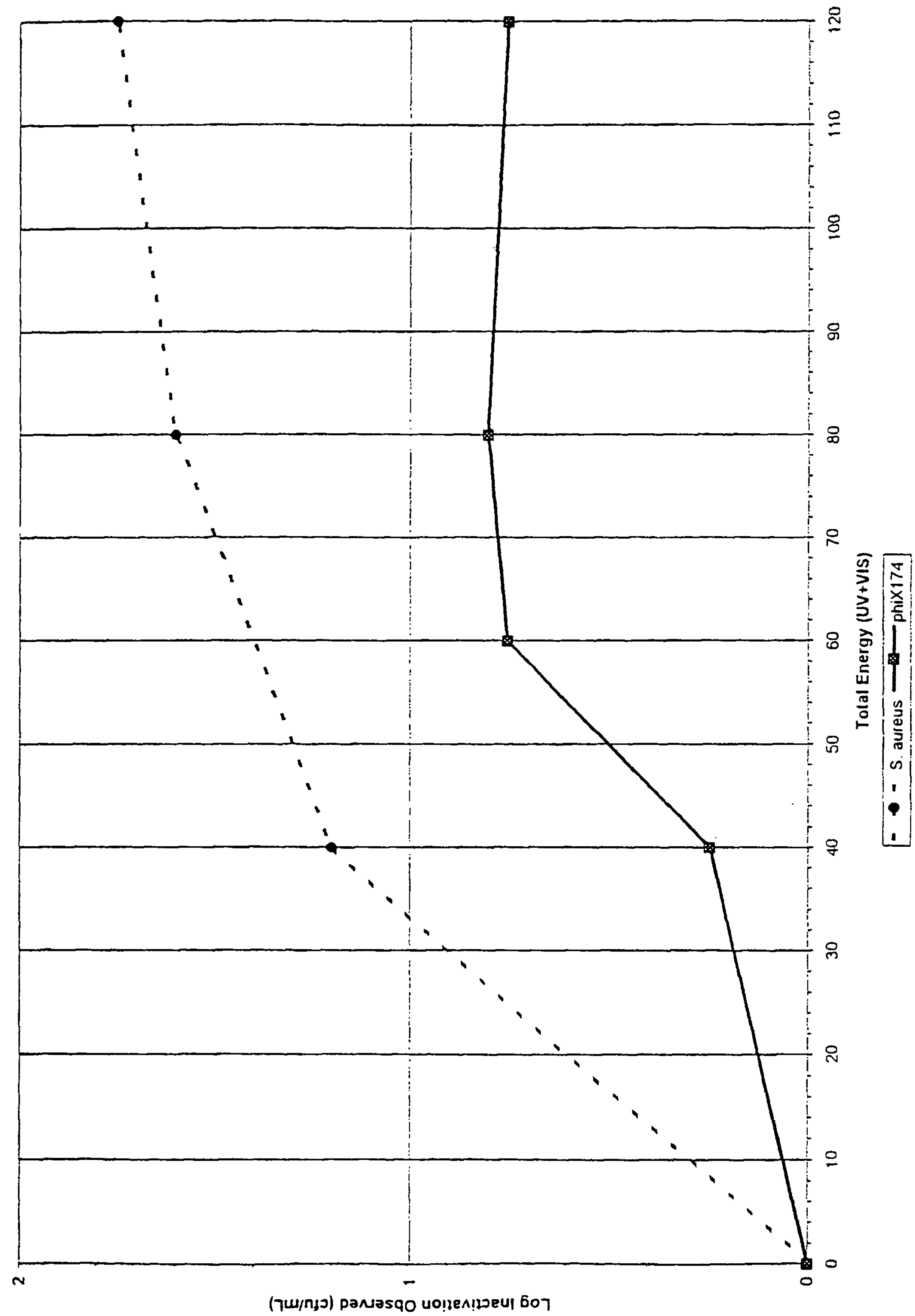
Log Inactivation Observed (cfu/mL)
0
1
2
Total Energy (UV+VIS)
0
10
20
30
40
50
60
70
80
90
100
110
120
S. aureus
phiX174

FIG. 14

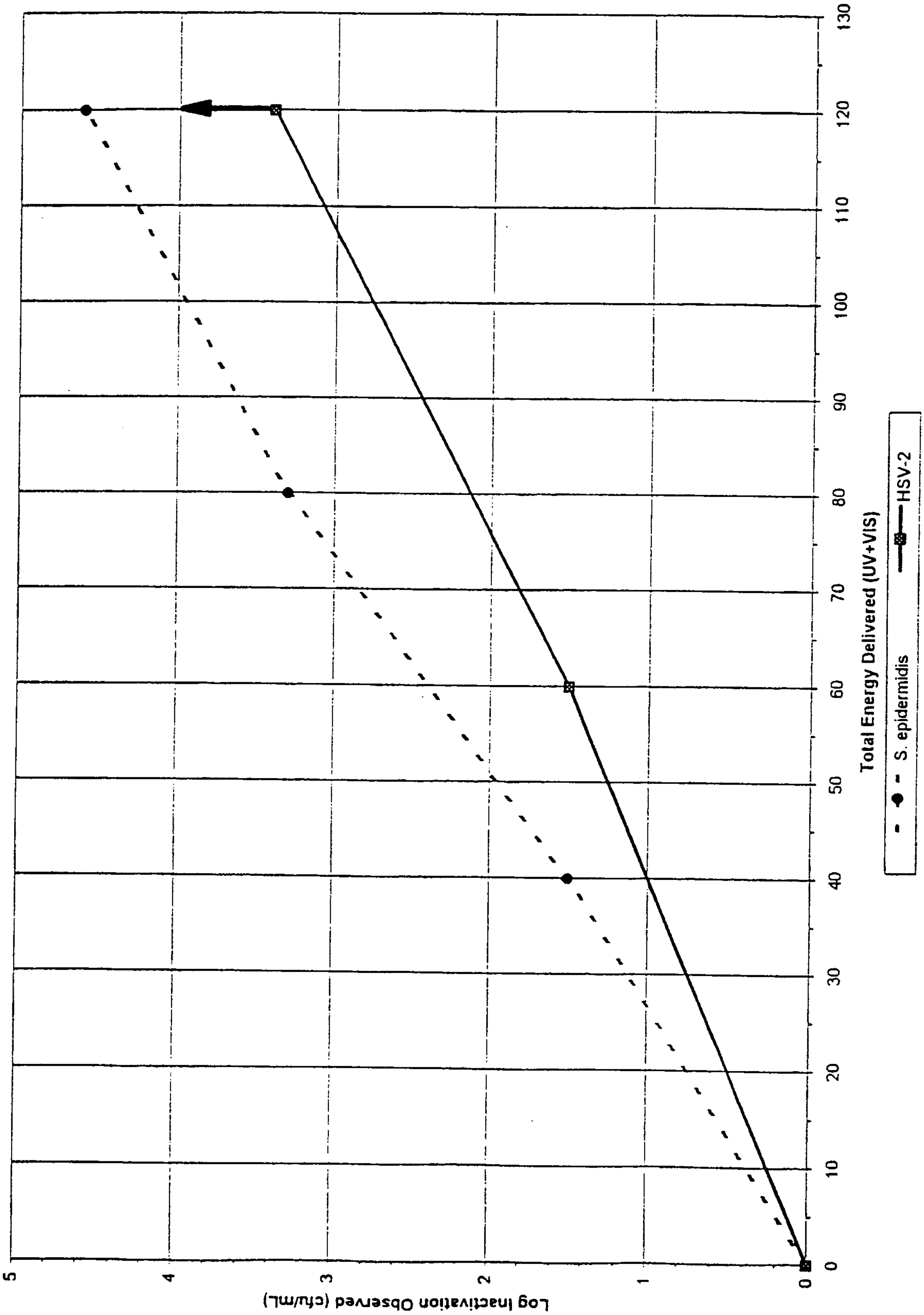
Log Inactivation Observed (cfu/mL)
0
1
2
3
4
5
Total Energy Delivered (UV+VIS)
0
10
20
30
40
50
60
70
80
90
100
110
120
130
S. epidermidis
HSV-2

FIG. 15

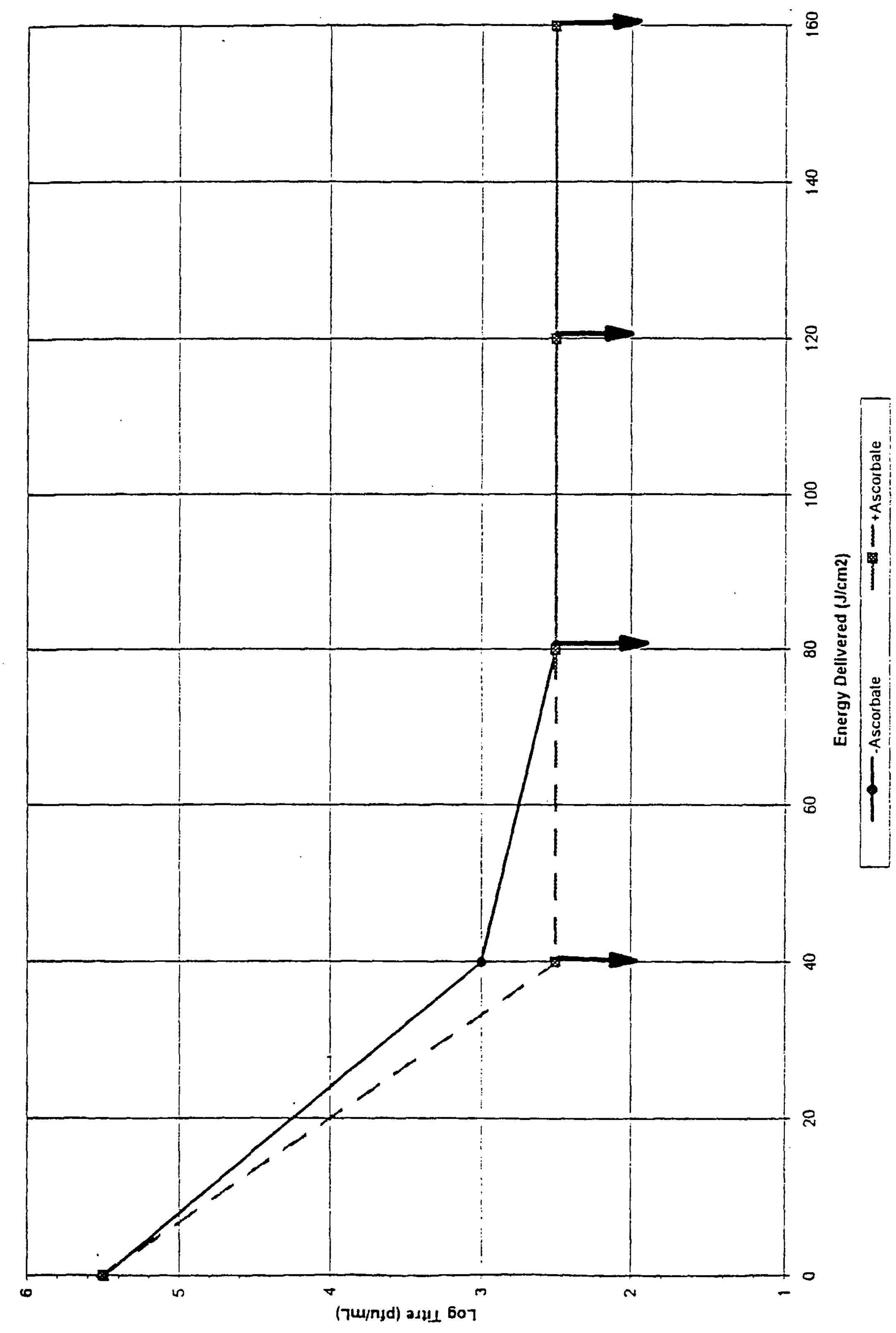
Log Titre (pfu/mL)
6
5
4
3
2
1
0
20
40
60
80
100
120
140
160
Energy Delivered (J/cm2)
-Ascorbate
+Ascorbate

FIG. 16

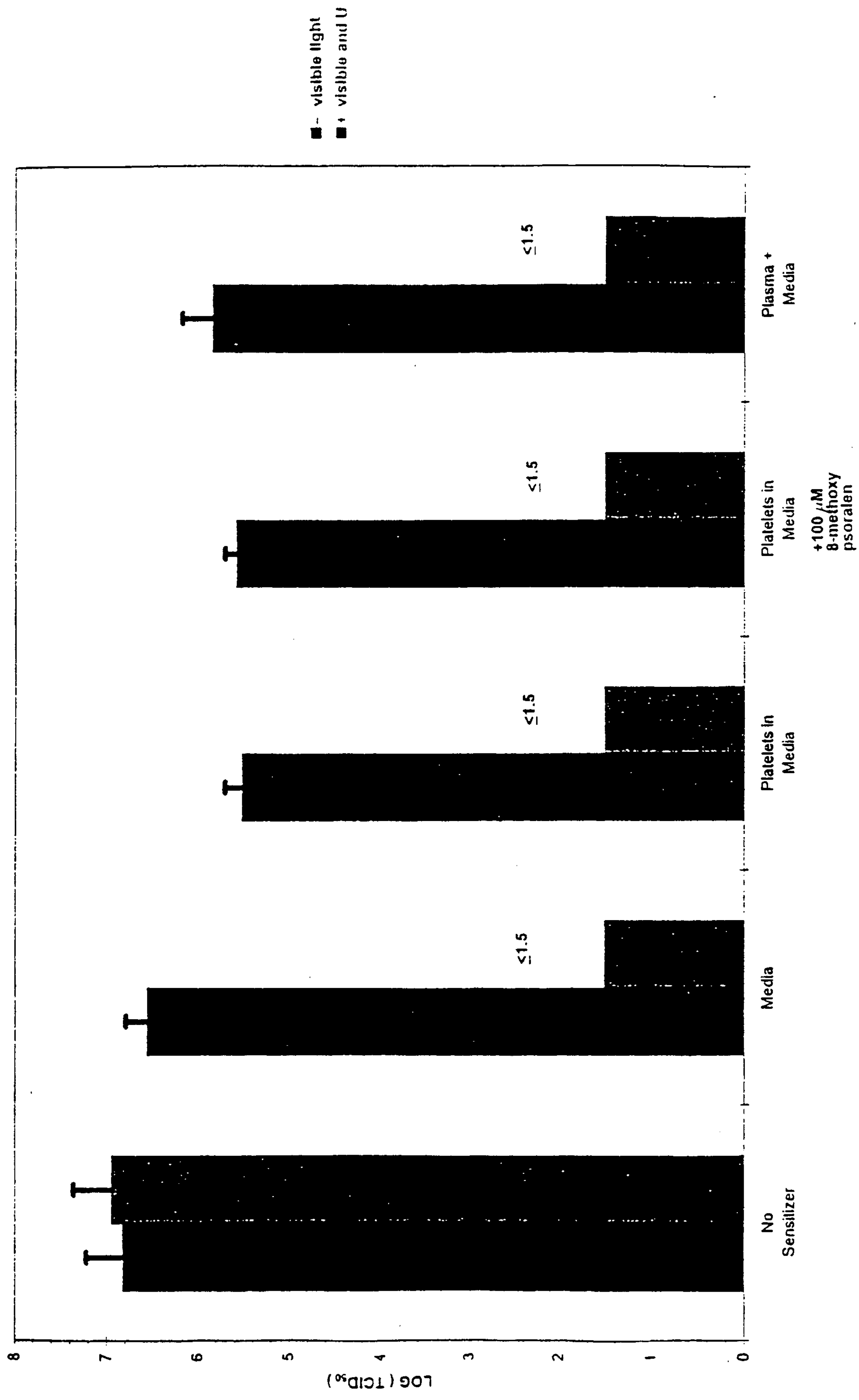
- visible light
+ visible and U
LOG ( TCID50 )
8
7
6
5
4
3
2
1
0
≤1.5
≤1.5
≤1.5
≤1.5
No Sensitizer
Media
Platelets in Media
Platelets in Media +100 μM 8-methoxy psoralen
Plasma + Media

FIG. 17

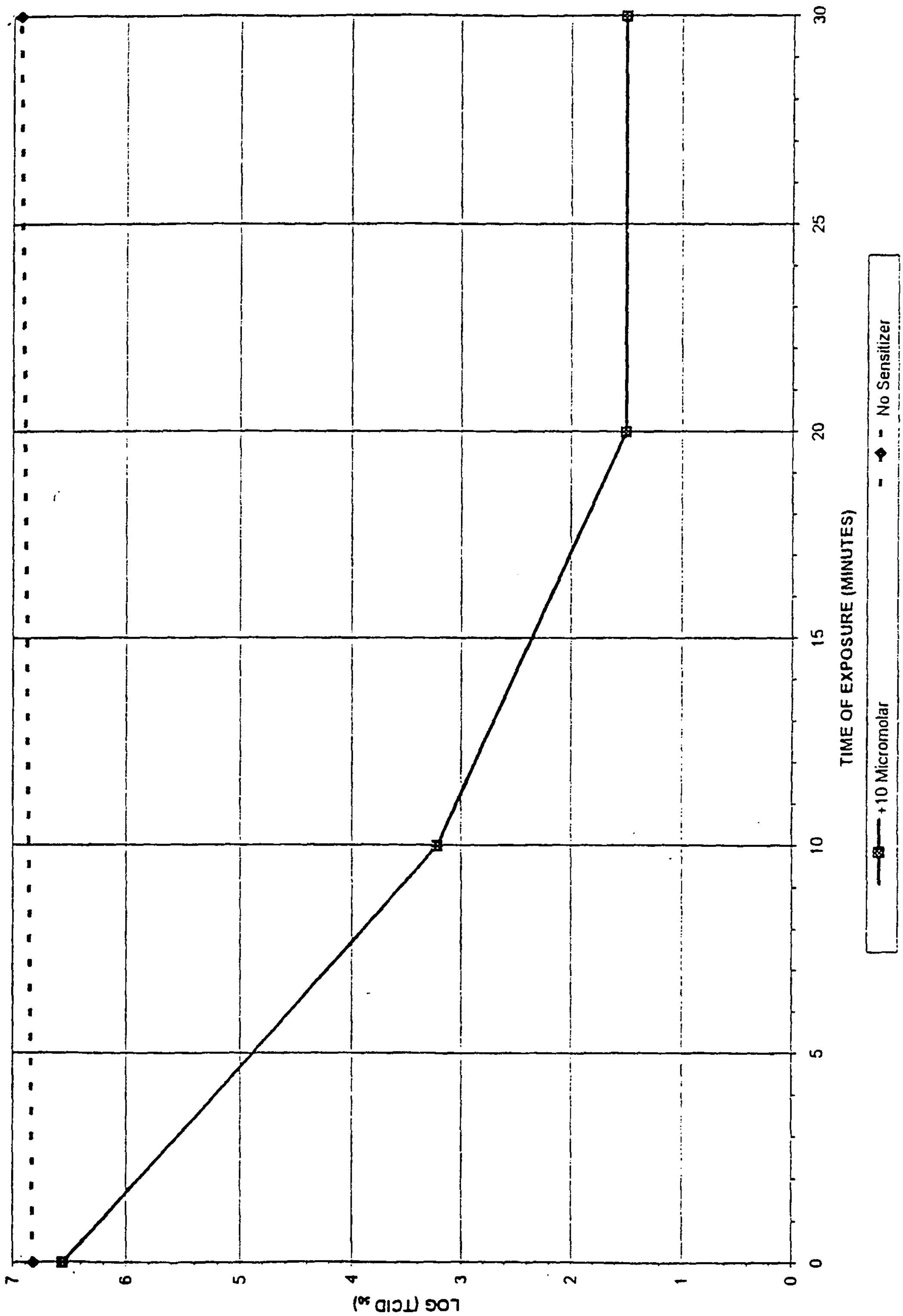

LOG (TCID 50)
7
6
5
4
3
2
1
0
0
5
10
15
20
25
30
TIME OF EXPOSURE (MINUTES)
+10 Micromolar
No Sensitizer


FIG. 18

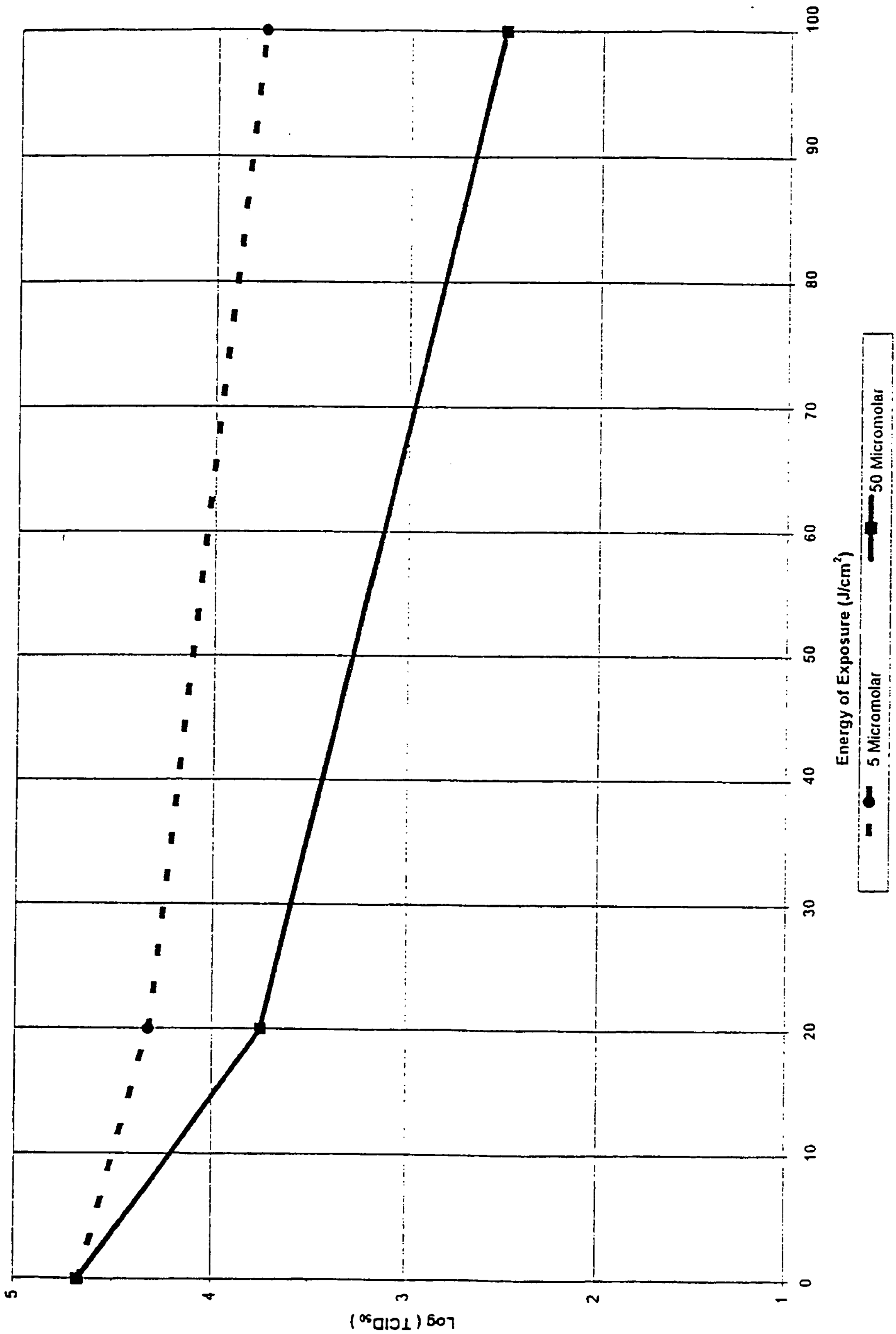

Log ( TCID50 )
5
4
3
2
1
0
10
20
30
40
50
60
70
80
90
100
Energy of Exposure (J/cm2)
5 Micromolar
50 Micromolar


FIG. 19

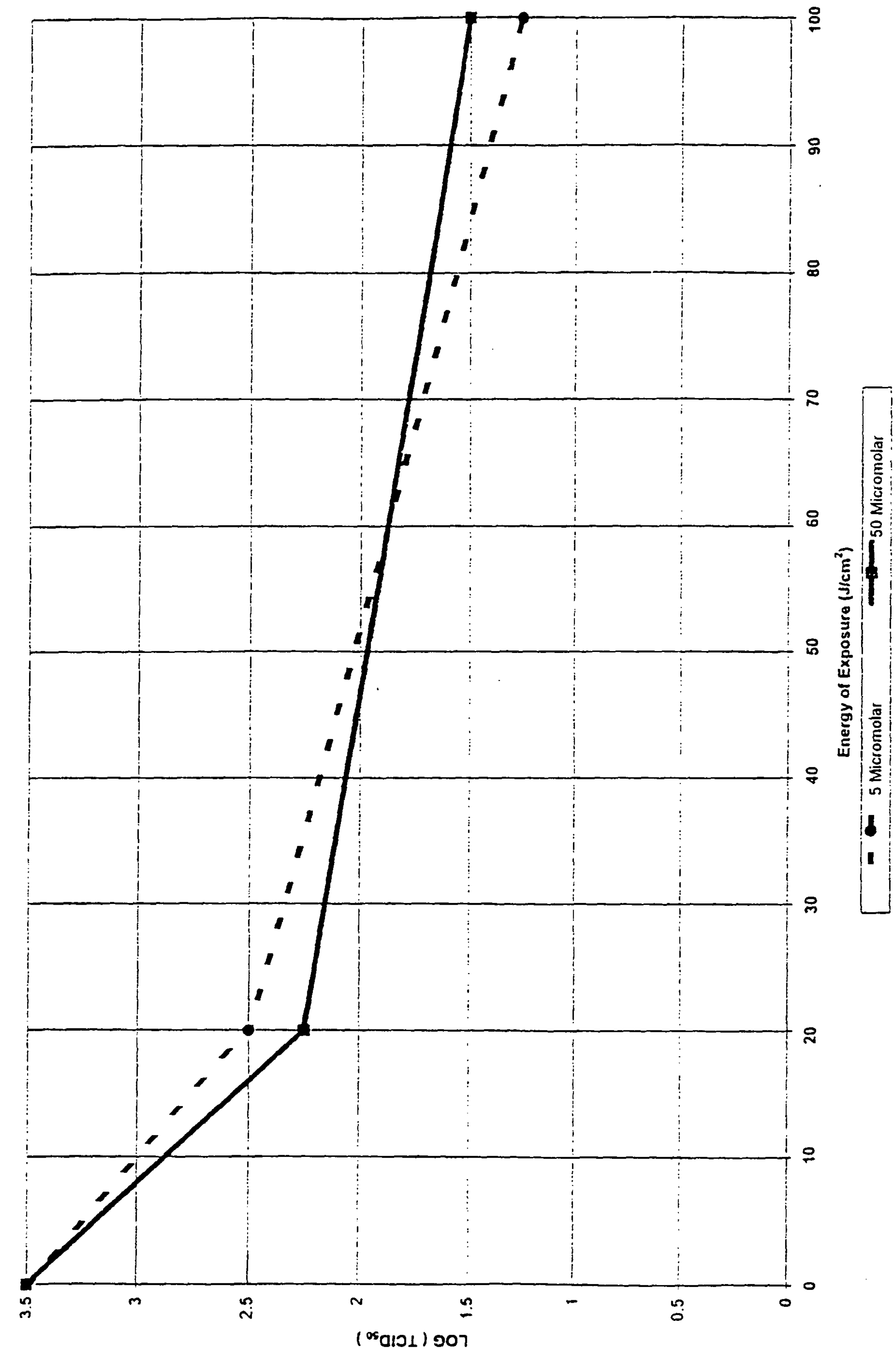
LOG ( TCID50 )
3.5
3
2.5
2
1.5
1
0.5
0
0
10
20
30
40
50
60
70
80
90
100
Energy of Exposure (J/cm²)
5 Micromolar
50 Micromolar

FIG. 20

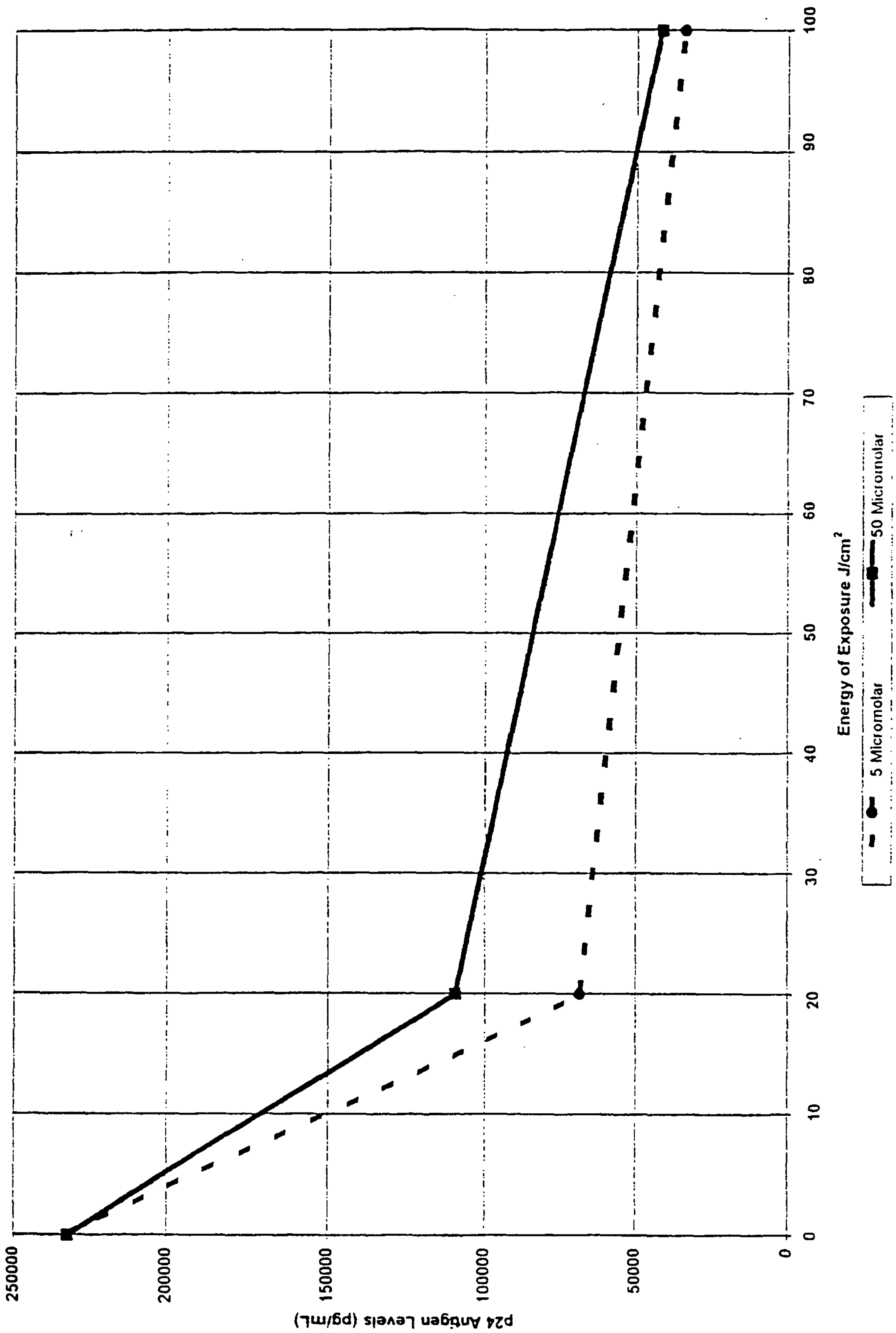
p24 Antigen Levels (pg/mL)
250000
200000
150000
100000
50000
0
Energy of Exposure J/cm²
0
10
20
30
40
50
60
70
80
90
100
5 Micromolar
50 Micromolar

FIG. 21

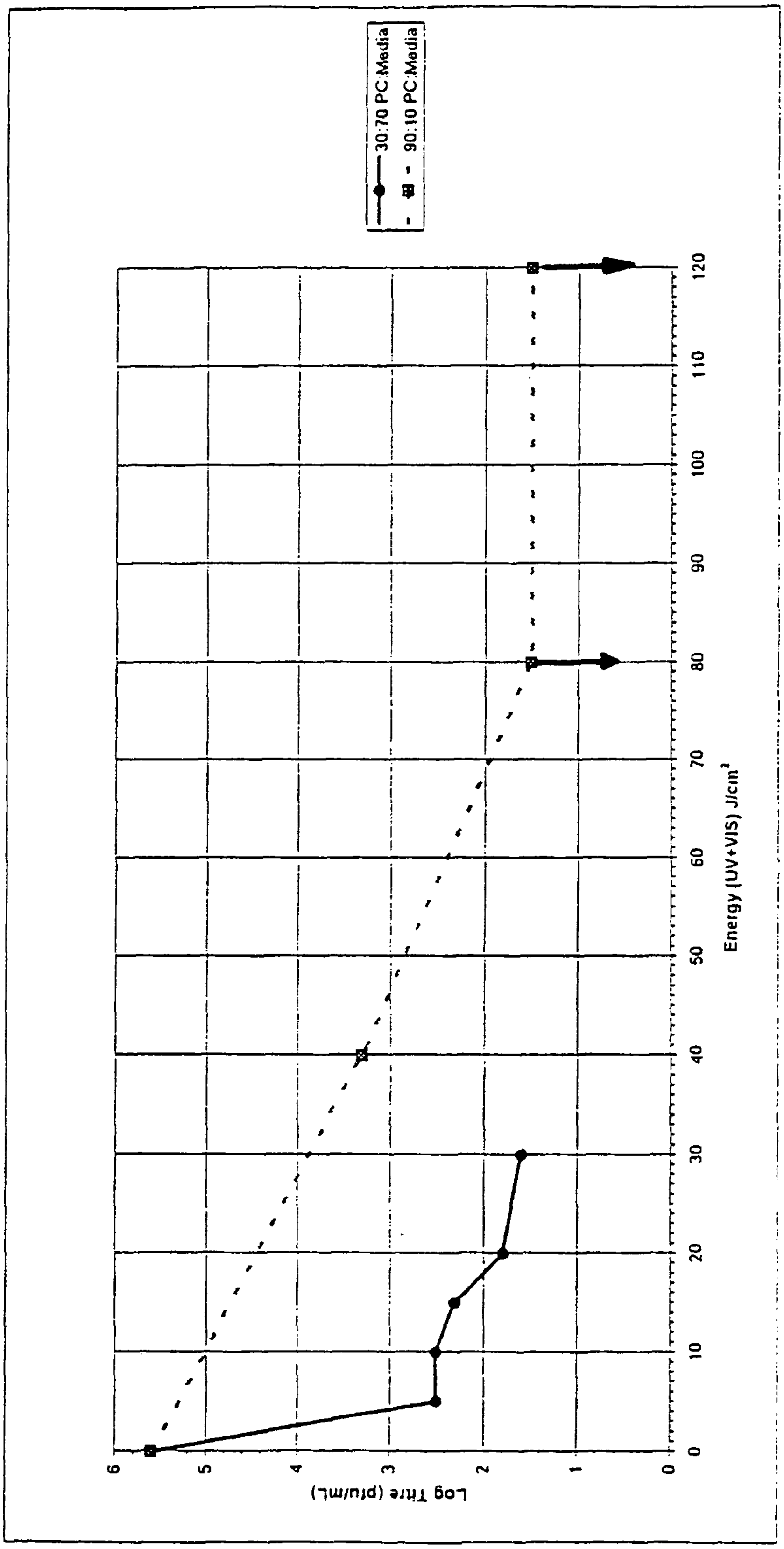
30:70 PC:Media
90:10 PC:Media
Log Titre (pfu/mL)
0
1
2
3
4
5
6
Energy (UV+VIS) J/cm²
0
10
20
30
40
50
60
70
80
90
100
110
120

FIG. 22

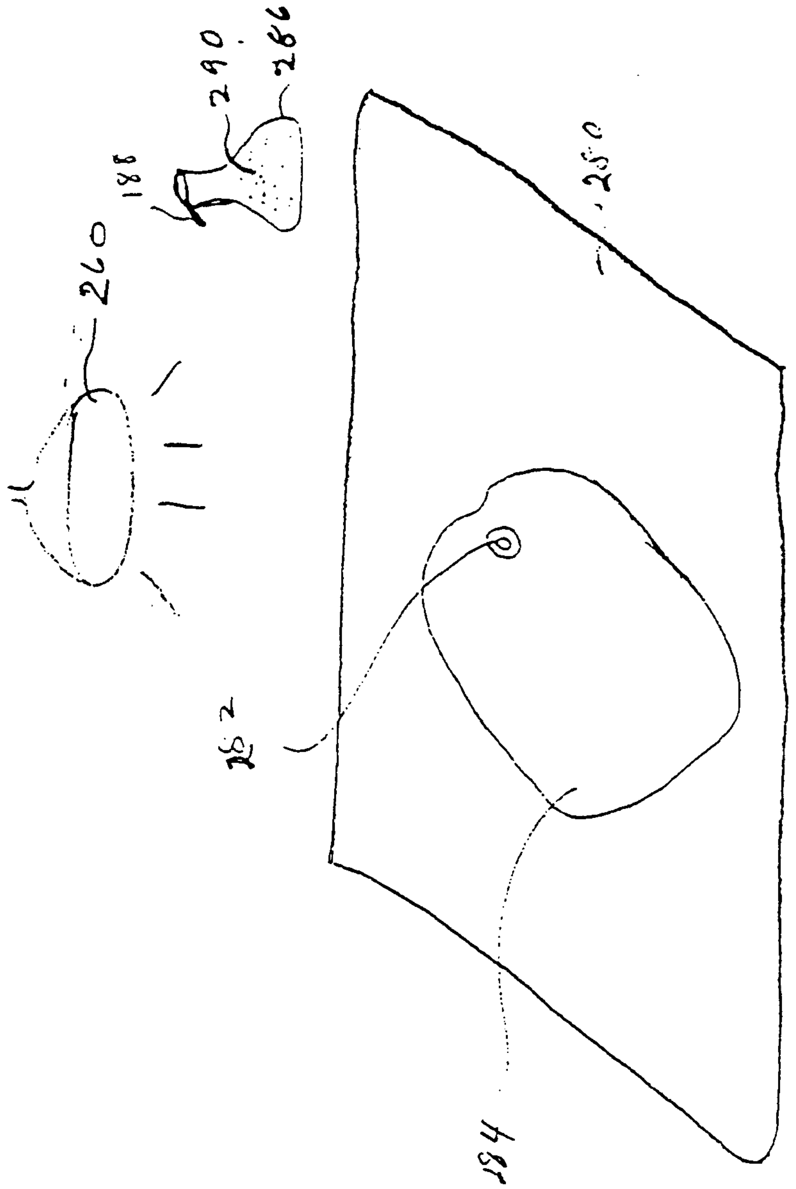


FIG. 23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 196515 A
- US 5607924 A
- US 5714328 A, Magda
- US 5041078 A, Matthews
- US 5545516 A, Wagner
- US 4915683 A
- US 5304113 A, Sieber
- US 4727027 A, Wiesehahn, G.P.
- US 5516629 A, Park
- US 5587490 A, Goodrich Jr., R.P.
- US 5418130 A, Platz
- US 5654443 A, Wollowitz
- US 5709991 A, Lin
- US 5120649 A
- US 5232844 A, Horowitz
- US 5360734 A, Chapman
- US 5342752 A, Platz
- US 5798238 A, Goodrich, Jr.
- US 4612007 A
- US 4683889 A, Edelson
- US 5290221 A, Wolfe, Jr.
- US 5536238 A, Bischof
- US 5653887 A
- US 4880788 A
- US 08924519 B
- WO 9911305 A


### Non-patent literature cited in the description


- **Goodrich, R.P. et al.** The Design and Development of Selective, Photoactivated Drugs for Sterilization of Blood Products. *Drugs of the Future,* 1997, vol. 22, 159-171
- **Tsugita, A et al.** Photosensitized inactivation of ribonucleic acids in the presence of riboflavin. *Biochimica et Biophysica Acta,* 1965, vol. 103, 360-363
- **Speck, W.T. et al.** Further Observations on the Photooxidation of DNA in the Presence of Riboflavin. *Biochimica et Biophysica Acta,* 1976, vol. 435, 39-44
- **Kuratomi, K. et al.** Studies on the Interactions between DNA and Flavins. *Biochimica et Biophysica Acta,* 1977, vol. 476, 207-217
- **Hoffmann, M.E. et al.** DNA Strand Breaks in Mammalian Cells Exposed to Light in the Presence of Riboflavin and Tryptophan. *Photochemistry and Photobiology,* 1979, vol. 29, 299-303
- **Piette, J. et al.** Production of Breaks in Single- and Double-Stranded Forms of Bacteriophage ΦX174 DNA by Proflavine and Light Treatment. *Photochemistry and Photobiology,* 1979, vol. 30, 369-378
- **Piette, J. et al.** Alteration of Guanine Residues during Proflavine Mediated Photosensitization of DNA. *Photochemistry and Photobiology,* 1981, vol. 33, 325-333
- **J. Cadet et al.** Mechanisms and Products of Photosensitized Degradation of Nucleic Acids and Related Model Compounds. *Israel J. Chem.,* 1983, vol. 23, 420-429
- **Korycka-Dahl, M. et al.** Photodegradation of DNA with Fluorescent Light in the Presence of Riboflavin, and Pholoprotection by Flavin Triplet-State Quenchers. *Biochimica et Biophysica Acta,* vol. 610, 229-234
- **Peak, J.G. et al.** DNA Breakage Caused by 334-nm Ultraviolet Light is Enhanced by Naturally Occurring Nucleic Acid Components and Nucleotide Coenzymes. *Photochemistry and Photobiology,* 1984, vol. 39, 713-716
- **Cadet et al.** *J. Chem.,* 1983, vol. 23, 420-429
- AABB Technical Manual. 1993
- **Gordon, A.J. ; Ford, R.A.** The Chemist's Companion: A Handbook of Practical Data, Techniques and References. John Wiley & Sons, 1972, 362-368